(19) Europäisches Patentamt / European Patent Office / Office européen des brevets

(11) EP 2 455 396 A1

(12) **EUROPEAN PATENT APPLICATION**
published in accordance with Art. 153(4) EPC

(43) Date of publication:
**23.05.2012 Bulletin 2012/21**

(21) Application number: **10799805.6**

(22) Date of filing: **12.07.2010**

(51) Int Cl.:
***C07K 14/705*** *(2006.01)* ***A61K 38/00*** *(2006.01)*
***A61P 37/06*** *(2006.01)* ***A61P 43/00*** *(2006.01)*

(86) International application number:
**PCT/JP2010/061749**

(87) International publication number:
**WO 2011/007747 (20.01.2011 Gazette 2011/03)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **16.07.2009 JP 2009168099**

(71) Applicant: **Otsuka Chemical Co., Ltd.**
**Osaka-shi, Osaka 540-0021 (JP)**

(72) Inventors:
- **KAJIHARA, Yasuhiro**
  **Toyonaka-shi**
  **Osaka 560-0054 (JP)**
- **TSUJI, Takashi**
  **Nagareyama-shi**
  **Chiba 270-0111 (JP)**
- **TEZUKA, Katsunari**
  **Tokushima-shi**
  **Tokushima 771-0093 (JP)**
- **YOSHIDA, Kenta**
  **Tokushima-shi**
  **Tokushima 771-0093 (JP)**
- **FUKAE, Kazuhiro**
  **Tokushima-shi**
  **Tokushima 771-0093 (JP)**

(74) Representative: **Wasner, Marita et al**
**Niizuma Wasner GmbH**
**Postfach 273**
**4125 Riehen 1 (CH)**

# (54) SUGAR CHAIN-ADDED AILIM EXTRACELLULAR DOMAIN AND METHOD FOR PRODUCING SAME

(57) Disclosed is a synthetic AILIM extracellular domain which has high ligand-binding ability and a quality sufficient for a pharmaceutical product. Also disclosed is a method for synthesizing the synthetic AILIM extracellular domain. Specifically disclosed is a sugar chain-added AILIM extracellular domain wherein a sugar chain is bound at a position corresponding to the 69-position of the amino acid sequence of human AILIM extracellular domain, said amino acid sequence being depicted in SEQ ID NO: 7, and no sugar chain is added at positions corresponding to the 3-position and the 90-position of the amino acid sequence.

EP 2 455 396 A1

SEQUENCE LISTING

<110> OTSUKA CHEMICAL CO., LTD.

<120> Glycosylated AILIM extracellular domain and the process of the same

<130> OCKP0904F

<150> JP2009-168099

<151> 2009-7-16

<160> 17

<170> PatentIn version 3.1

<210> 1
<211> 199
<212> PRT
<213> Homo sapiens

<220>
<223> human AILIM

<400> 1

```
Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Phe Trp Leu Pro
    130                 135                 140

Ile Gly Cys Ala Ala Phe Val Val Val Cys Ile Leu Gly Cys Ile Leu
145                 150                 155                 160
```

```
Ile Cys Trp Leu Thr Lys Lys Lys Tyr Ser Ser Ser Val His Asp Pro
                165                 170                 175

Asn Gly Glu Tyr Met Phe Met Arg Ala Val Asn Thr Ala Lys Lys Ser
            180                 185                 190

Arg Leu Thr Asp Val Thr Leu
        195


<210>  2
<211>  216
<212>  PRT
<213>  Rattus norvegicus

<220>
<223>  rat AILIM-1


<400>  2

Met Lys Pro Tyr Phe Ser Cys Val Phe Val Phe Cys Phe Leu Ile Lys
1               5                   10                  15

Leu Leu Thr Gly Glu Leu Asn Asp Leu Ala Asn His Arg Met Phe Ser
            20                  25                  30

Phe His Asp Gly Gly Val Gln Ile Ser Cys Asn Tyr Pro Glu Thr Val
        35                  40                  45

Gln Gln Leu Lys Met Gln Leu Phe Lys Asp Arg Glu Val Leu Cys Asp
    50                  55                  60

Leu Thr Lys Thr Lys Gly Ser Gly Asn Thr Val Ser Ile Lys Asn Pro
65                  70                  75                  80

Met Ser Cys Pro Tyr Gln Leu Ser Asn Asn Ser Val Ser Phe Phe Leu
                85                  90                  95

Asp Asn Ala Asp Ser Ser Gln Gly Ser Tyr Phe Leu Cys Ser Leu Ser
            100                 105                 110

Ile Phe Asp Pro Pro Pro Phe Gln Glu Lys Asn Leu Ser Gly Gly Tyr
        115                 120                 125

Leu Leu Ile Tyr Glu Ser Gln Leu Cys Cys Gln Leu Lys Leu Trp Leu
    130                 135                 140

Pro Val Gly Cys Ala Ala Phe Val Ala Ala Leu Leu Phe Gly Cys Ile
145                 150                 155                 160
```

```
Phe Ile Val Trp Phe Ala Lys Lys Lys Tyr Arg Ser Ser Val His Asp
                165                 170                 175

Pro Asn Ser Glu Tyr Met Phe Met Ala Ala Val Asn Thr Asn Lys Lys
            180                 185                 190

Ser Arg Leu Ala Gly Thr Ala Pro Leu Arg Ala Leu Gly Arg Gly Glu
        195                 200                 205

His Ser Ser Cys Gln Asp Arg Asn
    210                 215
```

<210> 3
<211> 200
<212> PRT
<213> Rattus norvegicus

<220>
<223> rat AILIM-2

<400> 3

```
Met Lys Pro Tyr Phe Ser Cys Val Phe Val Phe Cys Phe Leu Ile Lys
1               5                   10                  15

Leu Leu Thr Gly Glu Leu Asn Asp Leu Ala Asn His Arg Met Phe Ser
            20                  25                  30

Phe His Asp Gly Gly Val Gln Ile Ser Cys Asn Tyr Pro Glu Thr Val
        35                  40                  45

Gln Gln Leu Lys Met Gln Leu Phe Lys Asp Arg Glu Val Leu Cys Asp
    50                  55                  60

Leu Thr Lys Thr Lys Gly Ser Gly Asn Thr Val Ser Ile Lys Asn Pro
65                  70                  75                  80

Met Ser Cys Pro Tyr Gln Leu Ser Asn Asn Ser Val Ser Phe Phe Leu
                85                  90                  95

Asp Asn Ala Asp Ser Ser Gln Gly Ser Tyr Phe Leu Cys Ser Leu Ser
            100                 105                 110

Ile Phe Asp Pro Pro Pro Phe Gln Glu Lys Asn Leu Ser Gly Gly Tyr
        115                 120                 125

Leu Leu Ile Tyr Glu Ser Gln Leu Cys Cys Gln Leu Lys Leu Trp Leu
    130                 135                 140

Pro Val Gly Cys Ala Ala Phe Val Ala Ala Leu Leu Phe Gly Cys Ile
145                 150                 155                 160
```

```
Phe Ile Val Trp Phe Ala Lys Lys Lys Tyr Arg Ser Ser Val His Asp
                165                 170                 175

Pro Asn Ser Glu Tyr Met Phe Met Ala Ala Val Asn Thr Asn Lys Lys
            180                 185                 190

Ser Arg Leu Ala Gly Met Thr Ser
        195                 200


<210>  4
<211>  200
<212>  PRT
<213>  Mus musculus

<220>
<223>  mouse AILIM


<400>  4

Met Lys Pro Tyr Phe Cys His Val Gly Val Phe Cys Phe Leu Ile Arg
1               5                   10                  15

Leu Leu Thr Gly Glu Ile Asn Gly Ser Ala Asp His Arg Met Phe Ser
            20                  25                  30

Phe His Asn Gly Gly Val Gln Ile Ser Cys Lys Tyr Pro Glu Thr Val
        35                  40                  45

Gln Gln Leu Lys Met Arg Leu Phe Arg Glu Arg Glu Val Leu Cys Glu
    50                  55                  60

Leu Thr Lys Thr Lys Gly Ser Gly Asn Ala Val Ser Ile Lys Asn Pro
65                  70                  75                  80

Met Leu Cys Leu Tyr His Leu Ser Asn Asn Ser Val Ser Phe Phe Leu
                85                  90                  95

Asn Asn Pro Asp Ser Ser Gln Gly Ser Tyr Tyr Phe Cys Ser Leu Ser
            100                 105                 110

Ile Phe Asp Pro Pro Pro Phe Gln Glu Arg Asn Leu Ser Gly Gly Tyr
        115                 120                 125

Leu His Ile Tyr Glu Ser Gln Leu Cys Cys Gln Leu Lys Leu Trp Leu
    130                 135                 140

Pro Val Gly Cys Ala Ala Phe Val Val Val Leu Leu Phe Gly Cys Ile
145                 150                 155                 160
```

```
Leu Ile Ile Trp Phe Ser Lys Lys Lys Tyr Gly Ser Ser Val His Asp
                165                 170                 175

Pro Asn Ser Glu Tyr Met Phe Met Ala Ala Val Asn Thr Asn Lys Lys
            180                 185                 190

Ser Arg Leu Ala Gly Val Thr Ser
        195                 200


<210>  5
<211>  220
<212>  PRT
<213>  Homo sapiens

<220>
<223>  human CD28


<400>  5
Met Leu Arg Leu Leu Leu Ala Leu Asn Leu Phe Pro Ser Ile Gln Val
1               5                   10                  15

Thr Gly Asn Lys Ile Leu Val Lys Gln Ser Pro Met Leu Val Ala Tyr

            20                  25                  30

Asp Asn Ala Val Asn Leu Ser Cys Lys Tyr Ser Tyr Asn Leu Phe Ser
        35                  40                  45

Arg Glu Phe Arg Ala Ser Leu His Lys Gly Leu Asp Ser Ala Val Glu
    50                  55                  60

Val Cys Val Val Tyr Gly Asn Tyr Ser Gln Gln Leu Gln Val Tyr Ser
65                  70                  75                  80

Lys Thr Gly Phe Asn Cys Asp Gly Lys Leu Gly Asn Glu Ser Val Thr
                85                  90                  95

Phe Tyr Leu Gln Asn Leu Tyr Val Asn Gln Thr Asp Ile Tyr Phe Cys
            100                 105                 110

Lys Ile Glu Val Met Tyr Pro Pro Pro Tyr Leu Asp Asn Glu Lys Ser
        115                 120                 125

Asn Gly Thr Ile Ile His Val Lys Gly Lys His Leu Cys Pro Ser Pro
    130                 135                 140

Leu Phe Pro Gly Pro Ser Lys Pro Phe Trp Val Leu Val Val Val Gly
145                 150                 155                 160

Gly Val Leu Ala Cys Tyr Ser Leu Leu Val Thr Val Ala Phe Ile Ile
                165                 170                 175
```

Phe Trp Val Arg Ser Lys Arg Ser Arg Leu Leu His Ser Asp Tyr Met
180 185 190

Asn Met Thr Pro Arg Arg Pro Gly Pro Thr Arg Lys His Tyr Gln Pro
195 200 205

Tyr Ala Pro Pro Arg Asp Phe Ala Ala Tyr Arg Ser
210 215 220

<210> 6
<211> 223
<212> PRT
<213> Homo sapiens

<220>
<223> human CTLA4

<400> 6

Met Ala Cys Leu Gly Phe Gln Arg His Lys Ala Gln Leu Asn Leu Ala
1 5 10 15

Ala Arg Thr Trp Pro Cys Thr Leu Leu Phe Phe Leu Leu Phe Ile Pro
20 25 30

Val Phe Cys Lys Ala Met His Val Ala Gln Pro Ala Val Val Leu Ala
35 40 45

Ser Ser Arg Gly Ile Ala Ser Phe Val Cys Glu Tyr Ala Ser Pro Gly
50 55 60

Lys Ala Thr Glu Val Arg Val Thr Val Leu Arg Gln Ala Asp Ser Gln
65 70 75 80

Val Thr Glu Val Cys Ala Ala Thr Tyr Met Thr Gly Asn Glu Leu Thr
85 90 95

Phe Leu Asp Asp Ser Ile Cys Thr Gly Thr Ser Ser Gly Asn Gln Val
100 105 110

Asn Leu Thr Ile Gln Gly Leu Arg Ala Met Asp Thr Gly Leu Tyr Ile
115 120 125

Cys Lys Val Glu Leu Met Tyr Pro Pro Pro Tyr Tyr Leu Gly Ile Gly
130 135 140

Asn Gly Thr Gln Ile Tyr Val Ile Asp Pro Glu Pro Cys Pro Asp Ser

145 150 155 160

Asp Phe Leu Leu Trp Ile Leu Ala Ala Val Ser Ser Gly Leu Phe Phe
165 170 175

Tyr Ser Phe Leu Leu Thr Ala Val Ser Leu Ser Lys Met Leu Lys Lys
180 185 190

Arg Ser Pro Leu Thr Thr Gly Val Tyr Val Lys Met Pro Pro Thr Glu
195 200 205

Pro Glu Cys Glu Lys Gln Phe Gln Pro Tyr Phe Ile Pro Ile Asn
210 215 220

<210> 7
<211> 121
<212> PRT
<213> Homo sapiens

<220>
<223> human AILIM extracellular domain

<400> 7

Glu Ile Asn Gly Ser Ala Asn Tyr Glu Met Phe Ile Phe His Asn Gly
1 5 10 15

Gly Val Gln Ile Leu Cys Lys Tyr Pro Asp Ile Val Gln Gln Phe Lys
20 25 30

Met Gln Leu Leu Lys Gly Gly Gln Ile Leu Cys Asp Leu Thr Lys Thr
35 40 45

Lys Gly Ser Gly Asn Thr Val Ser Ile Lys Ser Leu Lys Phe Cys His
50 55 60

Ser Gln Leu Ser Asn Asn Ser Val Ser Phe Phe Leu Tyr Asn Leu Asp
65 70 75 80

His Ser His Ala Asn Tyr Tyr Phe Cys Asn Leu Ser Ile Phe Asp Pro
85 90 95

Pro Pro Phe Lys Val Thr Leu Thr Gly Gly Tyr Leu His Ile Tyr Glu
100 105 110

Ser Gln Leu Cys Cys Gln Leu Lys Phe
115 120

<210> 8
<211> 120
<212> PRT

<213> Homo sapiens

<220>
<223> human AILIM extracellular domain

<400> 8

Glu Ile Asn Gly Ser Ala Asn Tyr Glu Met Phe Ile Phe His Asn Gly
1 5 10 15

Gly Val Gln Ile Leu Cys Lys Tyr Pro Asp Ile Val Gln Gln Phe Lys
20 25 30

Met Gln Leu Leu Lys Gly Gly Gln Ile Leu Cys Asp Leu Thr Lys Thr
35 40 45

Lys Gly Ser Gly Asn Thr Val Ser Ile Lys Ser Leu Lys Phe Cys His
50 55 60

Ser Gln Leu Ser Asn Asn Ser Val Ser Phe Phe Leu Tyr Asn Leu Asp
65 70 75 80

His Ser His Ala Asn Tyr Tyr Phe Cys Asn Leu Ser Ile Phe Asp Pro
85 90 95

Pro Pro Phe Lys Val Thr Leu Thr Gly Gly Tyr Leu His Ile Tyr Glu
100 105 110

Ser Gln Leu Cys Cys Gln Leu Lys
115 120

<210> 9
<211> 120
<212> PRT
<213> Artificial

<220>
<223> glycosylated AILIM extracellular domain

<220>
<221> misc_feature
<222> (69)..(69)
<223> glycosylated Asn

<400> 9

Glu Ile Asn Gly Ser Ala Asn Tyr Glu Met Phe Ile Phe His Asn Gly
1 5 10 15

Gly Val Gln Ile Leu Cys Lys Tyr Pro Asp Ile Val Gln Gln Phe Lys
20 25 30

Met Gln Leu Leu Lys Gly Gly Gln Ile Leu Cys Asp Leu Thr Lys Thr

35 40 45

Lys Gly Ser Gly Asn Thr Val Ser Ile Lys Ser Leu Lys Phe Cys His
50 55 60

Ser Gln Leu Ser Asn Asn Ser Val Ser Phe Phe Leu Tyr Asn Leu Asp
65 70 75 80

His Ser His Ala Asn Tyr Tyr Phe Cys Asn Leu Ser Ile Phe Asp Pro
85 90 95

Pro Pro Phe Lys Val Thr Leu Thr Gly Gly Tyr Leu His Ile Tyr Glu
100 105 110

Ser Gln Leu Cys Cys Gln Leu Lys
115 120

<210> 10
<211> 32
<212> PRT
<213> Artificial

<220>
<223> Fragment D

<220>
<221> MOD_RES
<222> (28)..(28)
<223> Cys having blocking group Acm

<220>
<221> MOD_RES
<222> (29)..(29)
<223> Cys having blocking group Acm

<400> 10

Cys Asn Leu Ser Ile Phe Asp Pro Pro Pro Phe Lys Val Thr Leu Thr
1 5 10 15

Gly Gly Tyr Leu His Ile Tyr Glu Ser Gln Leu Cys Cys Gln Leu Lys
20 25 30

<210> 11
<211> 26
<212> PRT
<213> Artificial

<220>
<223> Fragment C

<220>
<221> MOD_RES
<222> (1)..(1)
<223> Thz having blocking group Boc

<220>
<221> MOD_RES
<222> (7)..(7)
<223> glycosylated Asn

<220>
<221> MOD_RES
<222> (26)..(26)
<223> Phe having benzyl thioester group

<400> 11

```
Cys His Ser Gln Leu Ser Asn Asn Ser Val Ser Phe Phe Leu Tyr Asn
1               5                   10                  15

Leu Asp His Ser His Ala Asn Tyr Tyr Phe
            20                  25
```

<210> 12
<211> 58
<212> PRT
<213> Artificial

<220>
<223> Fragment C+D

<220>
<221> MOD_RES
<222> (1)..(1)
<223> Thz having blocking group Boc

<220>
<221> MOD_RES
<222> (7)..(7)
<223> glycosylated Asn

<220>
<221> MOD_RES
<222> (54)..(54)
<223> Cys having blocking group Acm

<220>
<221> MOD_RES
<222> (55)..(55)
<223> Cys having blocking group Acm

<400> 12

```
Cys His Ser Gln Leu Ser Asn Asn Ser Val Ser Phe Phe Leu Tyr Asn
1               5                   10                  15

Leu Asp His Ser His Ala Asn Tyr Tyr Phe Cys Asn Leu Ser Ile Phe
            20                  25                  30

Asp Pro Pro Pro Phe Lys Val Thr Leu Thr Gly Gly Tyr Leu His Ile
        35                  40                  45
```

```
Tyr Glu Ser Gln Leu Cys Cys Gln Leu Lys
    50                  55


<210>  13
<211>  58
<212>  PRT
<213>  Artificial

<220>
<223>  Fragment C+D


<220>
<221>  MOD_RES
<222>  (7)..(7)
<223>  glycosylated Asn


<220>
<221>  MOD_RES
<222>  (54)..(54)
<223>  Cys having blocking group Acm


<220>
<221>  MOD_RES
<222>  (55)..(55)
<223>  Cys having blocking group Acm



<400>  13

Cys His Ser Gln Leu Ser Asn Asn Ser Val Ser Phe Phe Leu Tyr Asn
1               5                   10                  15


Leu Asp His Ser His Ala Asn Tyr Tyr Phe Cys Asn Leu Ser Ile Phe
            20                  25                  30


Asp Pro Pro Pro Phe Lys Val Thr Leu Thr Gly Gly Tyr Leu His Ile
        35                  40                  45


Tyr Glu Ser Gln Leu Cys Cys Gln Leu Lys
    50                  55


<210>  14
<211>  20
<212>  PRT
<213>  Artificial

<220>
<223>  Fragment B


<220>
<221>  MOD_RES
<222>  (20)..(20)
<223>  Phe having propane thioester group
```

<400> 14

Cys Asp Leu Thr Lys Thr Lys Gly Ser Gly Asn Thr Val Ser Ile Lys
1               5                   10                  15

Ser Leu Lys Phe
            20

<210> 15
<211> 42
<212> PRT
<213> Artificial

<220>
<223> Fragment A

<220>
<221> MOD_RES
<222> (22)..(22)
<223> Cys having blocking group Acm

<220>
<221> MOD_RES
<222> (42)..(42)
<223> Leu having thiophenyl ester group

<400> 15

Glu Ile Asn Gly Ser Ala Asn Tyr Glu Met Phe Ile Phe His Asn Gly
1               5                   10                  15

Gly Val Gln Ile Leu Cys Lys Tyr Pro Asp Ile Val Gln Gln Phe Lys
            20                  25                  30

Met Gln Leu Leu Lys Gly Gly Gln Ile Leu
        35                  40

<210> 16
<211> 62
<212> PRT
<213> Artificial

<220>
<223> Fragment A+B

<220>
<221> MOD_RES
<222> (22)..(22)
<223> Cys having blocking group Acm

<220>
<221> MOD_RES
<222> (62)..(62)
<223> Phe having propane thioester group

<400> 16

Glu Ile Asn Gly Ser Ala Asn Tyr Glu Met Phe Ile Phe His Asn Gly
1               5                   10                  15

Gly Val Gln Ile Leu Cys Lys Tyr Pro Asp Ile Val Gln Gln Phe Lys
            20                  25                  30

Met Gln Leu Leu Lys Gly Gly Gln Ile Leu Cys Asp Leu Thr Lys Thr
        35                  40                  45

Lys Gly Ser Gly Asn Thr Val Ser Ile Lys Ser Leu Lys Phe
    50                  55                  60

<210> 17
<211> 120
<212> PRT
<213> Artificial

<220>
<223> Fragment A+B+C+D

<220>
<221> MOD_RES
<222> (22)..(22)
<223> Cys having blocking group Acm

<220>
<221> MOD_RES
<222> (69)..(69)
<223> glycosylated Asn

<220>
<221> MOD_RES
<222> (116)..(116)
<223> Cys having blocking group Acm

<220>
<221> MOD_RES
<222> (117)..(117)
<223> Cys having blocking group Acm

<400> 17

Glu Ile Asn Gly Ser Ala Asn Tyr Glu Met Phe Ile Phe His Asn Gly
1               5                   10                  15

Gly Val Gln Ile Leu Cys Lys Tyr Pro Asp Ile Val Gln Gln Phe Lys
            20                  25                  30

Met Gln Leu Leu Lys Gly Gly Gln Ile Leu Cys Asp Leu Thr Lys Thr

```
        35                  40                  45

Lys Gly Ser Gly Asn Thr Val Ser Ile Lys Ser Leu Lys Phe Cys His
    50                  55                  60

Ser Gln Leu Ser Asn Asn Ser Val Ser Phe Phe Leu Tyr Asn Leu Asp
65                  70                  75                  80

His Ser His Ala Asn Tyr Tyr Phe Cys Asn Leu Ser Ile Phe Asp Pro
                85                  90                  95

Pro Pro Phe Lys Val Thr Leu Thr Gly Gly Tyr Leu His Ile Tyr Glu
            100                 105                 110

Ser Gln Leu Cys Cys Gln Leu Lys
        115                 120
```

## Description

Technical Field

The present invention relates to an oligosaccharide chain-added extracellular domain of AILIM and a method for producing the same.

Background Art

AILIM (activation-inducible lymphocyte immunomediatory molecule) is expressed on T cell surface and is one of costimulatory receptors having the function of activating T cells. AILIM is also known as the name of ICOS (inducible-costimulator) or AILIM/ICOS, or the like (see e.g., Patent Literature 1 and Non Patent Literature 1).

The first signals mediated by an antigenic peptide bound to a major histocompatibility complex (MHC) on antigen-presenting cells and a T cell antigen receptor/CD3 complex on T cell surface as well as the second signals mediated by costimulatory molecules are essential for the activation of antigen-specific T cells. Costimulatory receptors are T cell receptors that bind to costimulatory molecules on antigen-presenting cells. Integrin family, immunoglobulin superfamily, and TNF/TNF receptor family have been reported so far as the costimulatory receptors. AILIM has been shown to be the third molecule following CD28 and CTLA4 in the CD28/CTLA4 family belonging to the immunoglobulin superfamily.

Ligands of CD28 and CTLA4 (costimulatory molecules) are CD80 (B7-1) and CD86 (B7-2), respectively, and B7h, B7RP-1, and GL50 have been reported so far as AILIM ligands (costimulatory molecules) (see e.g., Non Patent Literatures 2 to 4).

Various attempts have been made in recent years to regulate the interactions between costimulatory molecules and costimulatory receptors and thereby regulate immune response *in vivo.*

CD28 is a molecule that strongly promotes the functions of T cells indispensable to antigen-specific immune response, whereas CTLA4 is a molecule that inhibits the activation of T cells. AILIM has been found to be a regulatory molecule that produces signals promoting the activation of T cells, as in CD28 (see e.g., Non Patent Literature 5).

CD28 is constitutively expressed on T cells, whereas AILIM is expressed at a low level on resting T cells.

The expression of AILIM is induced as the cells are activated, as in CTLA4. This shows that the initial stage of immune response is regulated mainly by the pathway involving CD28 and the CD40 ligand, whereas AILIM is deeply involved in the control of secondary reaction in the immunoreaction. Moreover, AILIM does not increase the production of interleukin 2, but induces the synthesis of interleukin 10, a factor inducing B cell differentiation. Furthermore, its expression is induced in response to inflammation or pathological conditions.

In addition, a phenomenon is also seen in which AILIM-expressing cells are localized in inflammatory sites.

These facts can suggest that inflammation or disease in which AILIM is highly involved may be controlled specifically by regulating the interaction between AILIM and its ligand rather than by controlling the overall functions of T cells.

In cell transfer from a donor differing in transplantation antigen, such as bone marrow transplantation, there occur the recognition of alloantigens and the activation of donor T cells by signals from costimulatory receptors, resulting in induction of GvHD (graft-versus-host disease). Therefore, a method has been proposed by which only signals from costimulatory molecules are blocked to induce immunological unresponsiveness (anergy) so that GvHD is inhibited. This method allows experimental analysis by *in vitro* mixed lymphocyte reaction (MLR) through which T cells from a donor are cocultured with antigen-presenting cells derived from a donor differing in transplantation antigen therefrom. For example, upon administration of soluble CTLA4, CD28-mediated signal transduction is inhibited to thereby inhibit MLR such that anergy is induced (see e.g., Non Patent Literatures 6 and 7).

Furthermore, in MLR using T cells activated by CD3 stimulation, soluble AILIM has been confirmed to have reaction inhibitory effect equivalent to that of soluble CTLA4. Thus, the applicability of AILIM to medical transplantation has been expected (see e.g., Non Patent Literature 8). The soluble AILIM used in the literature was expressed as a fusion protein of residues 1 (Met) to 135 (Leu) in AILIM protein with the Fc domain of human IgG1.

AILIM is a type I membrane protein containing a signal sequence (20 amino acid residues), an extracellular domain (human: 121 amino acid residues; rat and mouse: 122 amino acid residues), a transmembrane domain (21 amino acid residues), and an intracellular domain (32 amino acid residues) in this order from the N-terminus.

The natural form of human AILIM is glycosylated at asparagine residues 23, 89, and 110 in the extracellular domain. It has been reported that a lack of glycosylation at residue 89, among them, significantly reduces the ability to bind to the ligand B7h on antigen-presenting cells.

Thus, use of AILIM as pharmaceuticals requires obtaining soluble AILIM glycosylated at least at residue 89, in high quality without variations in oligosaccharide chain structure among production lots. Attempts for producing soluble AILIM have been made by gene recombination and chemical synthesis.

Patent Literature 1 discloses expression of a fusion protein of the extracellular domain of rat AILIM with IgG

Fc using an expression system. Patent Literature 3 discloses that a fusion protein of the extracellular domain of mouse AILIM with IgG Fc exhibits immunosuppressive effect in heart transplantation (see e.g., Patent Literature 3). Though Patent Literatures 1 and 3 do not make particular mention about oligosaccharide chains bound to the fusion proteins, the fusion proteins are considered to be inhomogeneously glycosylated with several types of oligosaccharide chains at N-glycosylated consensus sequences (Asn-Xaa-Ser or Thr) by post-translational modification, as long as they are expressed in an expression system. Such a protein having variations in oligosaccharide chain is unsuitable for use as pharmaceuticals.

Moreover, it has also been reported that a fusion protein of residues 1 to 141 of AILIM having an oligosaccharide chain only at residue 89, with IgG Fc was expressed using an expression system. However, in this method, Asn residues 23 and 110 must be substituted by other amino acids to prevent glycosylation at these residues. Thus, its peptide skeleton cannot have the same amino acid sequence as the natural one. Moreover, since the fusion protein is expressed in an expression system, they are inhomogeneously glycosilated. Thus, it is difficult to obtain quality sufficient for use as pharmaceuticals.

In these examples, the extracellular domain of AILIM is expressed as a fusion protein with IgG Fc, probably because, for example, the expressed protein can be purified in the same way as antibody; use of a human IgG Fc sequence may hardly cause antigenicity; and improvement in activity can be expected because dimerization can be achieved via the -S-S- bond in the antibody hinge region.
However, a larger molecule results in larger steric hindrance, which might reduce affinity for the ligand.

Meanwhile, native chemical ligation (NCL) method has heretofore been known as a method for synthesizing glycoproteins (see e.g., Patent Literature 2). This method is a method by which a peptide fragment having Cys as an N-terminal amino acid and a peptide fragment having a thioester at the C-terminus are linked to obtain a larger peptide chain. In this method, glycoproteins can be synthesized by using oligosaccharide chain added peptide fragments. Each fragment can be synthesized, for example, by a solid-phase synthesis method for elongating amino acids one by one. During the course of synthesis, oligosaccharide chain added asparagines having homogeneous oligosaccharide chains can be bound thereto instead of the amino acids to thereby obtain oligosaccharide chain added fragments having homogeneous amino acid sequences and oligosaccharide chain structures. The NCL method using such oligosaccharide chain added fragments can be performed to thereby obtain homogeneous glycoproteins that are free from variations among production lots or the like and may be used as pharmaceuticals.

When the protein of interest is synthesized by the NCL method, fragments are first designed so that the protein of interest is divided on the N-terminal side of one or more Cys residue(s). Then, each fragment is synthesized, and the C-terminus of the fragment to be linked on the N-terminal side is thioesterified. Then, the thioester is reacted with the N-terminal Cys residue of the fragment to be linked on the C-terminal side to link these fragments.
For example, the synthesis of the protein of interest by dividing it into fragments W, X, Y, and Z in this order from the N-terminus will be described as an example. In the description below, the fragment X linked to the fragment Y is referred to as a fragment X + Y.
The fragments X, Y, and Z are designed so that each of them has a Cys residue at the N-terminus.
The fragment Z is synthesized so that it has a carboxyl group at the C-terminus. The respective C-termini of the fragments W to Y thus synthesized are thioesterified. In this way, the fragment Z and the fragment Y are first linked by the NCL method. Subsequently, the fragment X and the fragment Y + Z and then the fragment W and the fragment X + Y + Z are linked so that one of the fragments to be linked inevitably has a carboxyl group at the C-terminus while the other fragment has a thioester at the C-terminus. Thus, the protein of interest can be synthesized efficiently without reversing the order of linking.

Thus, according to a standard method, the present inventors have divided an extracellular domain (residues 21 to 140) of AILIM into 5 fragments with the N-terminal sides of the Cys residues as boundaries, and synthesized each fragment. Then, the present inventors have attempted to link them by the NCL method. Specifically, the extracellular domain of AILIM was divided into a fragment 1 (residues 21 to 41), a fragment 2 (residues 42 to 62), a fragment 3 (residues 63 to 82), a fragment 4 (residues 83 to 108), and a fragment 5 (residues 109 to 140). Each fragment was synthesized by a solid-phase synthesis method. Then, the fragments were sequentially linked by the NCL method from the fragments 5 and 4. The synthesis scheme is shown in Figure 5 (Route A).
However, in this method, the oligosaccharide chain added fragment 4 is linked at the initial stage. Therefore, a loss of fragments in any of the subsequent steps leads to the consumption of expensive oligosaccharide chains. Thus, this method is unsuitable for volume production.

Next, the present inventors have performed the total synthesis of the extracellular domain of AILIM by an alternative route (Route B) for the purpose of reducing the consumption of oligosaccharide chain added fragments. The synthesis scheme is shown in Figure 6. In this method, both the fragments 2 and 3 to be linked and both the fragments 2 + 3 and 1 to be linked have a thioesterified C-terminus. Therefore, a KCL method (kinetically controlled ligation; see e.g., Non Patent Literature 9), which uses thioesters differing in eliminability, was used instead of the NCL method. Specifically, the fragments 1 and 2 each had a thiophenyl ester, which is a highly eliminable thioester, at the C-terminus,

and the fragment 3 had a propane thioester at the C-terminus. According to the KCL method, the compound of interest can be obtained in relatively large amounts based on difference in reaction rate even though each of the fragments to be linked has a thioester at the C-terminus. However, its conditions are difficult to study, and by-products are also formed in large amounts. Particularly, when the fragments 2 and 3 were linked, cyclic by-products formed as a result of intramolecular nucleophilic attack on the C-terminal thioester by the thiol group in the side chain of Cys in the fragment 2 + 3 as well as by-products formed as a result of nucleophilic attack on the thioester in the fragment 2 thereby were confirmed. In fact, as a result of performing the KCL method twice, the fragment 1 + 2 + 3 of interest was obtained in insufficient amounts as the synthesis proceeded.

Subsequently, the present inventors have performed the total synthesis of the extracellular domain of AILIM by an alternative route (Route C) for decreasing the number of runs of the KCL method. The synthesis scheme is shown in Figure 7. However, in this method as well, the fragment 2 + 3 + 4 + 5 of interest was not sufficiently obtained in the step of linking the fragment 2 + 3 and the fragment 4 + 5. Thus, it fell short of linking the fragment 1.
In this method as well, the cyclization of the fragment 2 + 3 was seen, and this by-product seemed to hinder the subsequent reaction. Thus, the present inventors have conducted studies using varying types of thiol catalysts; however, failed to find a thiol catalyst that prevents side reaction and efficiently promotes the NCL method. Furthermore, the present inventors have studied a method by which the thiol group in the Cys residue (residue 63) in the linking portion of the fragment 2 + 3 is protected to thereby prevent side reaction; however, reached a conclusion that it is difficult to selectively protect Cys after linking by the NCL method, thus Route C is also unsuitable for the total synthesis.

Furthermore, the present inventors have studied a new route (Route D) for preventing side reaction (particularly, cyclization) in the synthesis of the fragment 2 + 3. The synthesis scheme is shown in Figure 8. In Route D, first, a 41-residue fragment 2 + 3 was continuously synthesized by a Boc solid-phase synthesis method. In this synthesis, Cys protected with a protective group was used to thereby obtain a fragment 2 + 3 containing a protected Cys residue at residue 63. Subsequently, the fragment 2 + 3 was linked to a fragment 4 + 5 by the NCL method. Finally, a fragment 1 was linked thereto by the NCL method.
In this method, the cyclization of the fragment 2 + 3 by the thiol group of its Cys residue could be reduced considerably. However, the synthesis based on the Boc method resulted in insufficient yields of the fragment 2 + 3. Moreover, the fragment 2 + 3 + 4 + 5 was also obtained in insufficient amounts due to the presence of a large number of side reactions other than those derived from the fragment 2 + 3. In fact, it fell short of linking the fragment 1.

Thus, the synthesis of oligosaccharide chain added polypeptides by several runs of ligation methods requires optimizing a large number of conditions such as the design and synthesis of fragments, the order of ligation, the selection of reagents, and the addition of protective groups. As described above, the present inventors have previously studied the synthesis of an oligosaccharide chain-added extracellular domain of AILIM by various routes or conditions, but failed to achieve the total synthesis.

Citation List

Patent Literature

Patent Literature 1: Pamphlet of International Publication No. WO 98/38216
Patent Literature 2: Pamphlet of International Publication No. WO 96/34878
Patent Literature 3: Japanese Patent Laid-Open No. 2003-277293

Non Patent Literature

Non Patent Literature 1: Hutloff A, et al.: Nature (1999) 397: 263-266
Non Patent Literature 2: Yoshinaga SK, et al.: Nature (1999) 402: 827-832
Non Patent Literature 3: Swallow MM, et al.: Immunity (1999) 11: 423-432
Non Patent Literature 4: Ling V, et al.: J Immunol (2000) 164: 1653-1657
Non Patent Literature 5: Sakamoto S, et al.: HYBRIDOMA AND HYBRIDOMICS (2001) 20: 293-303
Non Patent Literature 6: Tan P, et al.: J Exp Med (1993) 177: 165-173
Non Patent Literature 7: Gribben JG, et al.: Blood (1996) 87: 4887-4893
Non Patent Literature 8: Aicher A, et al.: J Immunol (2000) 164: 4689-4696
Non Patent Literature 9: Bang D, et al.: Angew. Chem. Int. Ed. (2006) 45: 3985-3988

## Summary of Invention

### Technical Problem

The extracellular domain of AILIM specifically controls inflammation or disease in which AILIM is highly involved, and has been expected to be applicable as immunosuppressive agents or the like in medical transplantation.
Thus, an object of the present invention is to provide an extracellular domain of AILIM that has the high ability to bind to its ligand and has quality available as pharmaceuticals, and to provide a method for producing the same.

### Solution to Problem

The present inventors have conducted studies in light of the object and consequently found that: a fragment 1 + 2 + 3 and a fragment 4 + 5 can each be obtained in sufficient amounts by (i) continuously synthesizing, by a solid-phase synthesis method, fragments 1 and 2, which have conventionally been synthesized individually and linked by a ligation method, (ii) introducing protective groups to Cys residues unnecessary for ligation contained in the fragment 1 + 2 and a fragment 5, (iii) linking the fragment 1 + 2 to a fragment 3 by a KCL method, and (iv) linking fragments 4 and 5 by an NCL method; and the total synthesis can be achieved by (v) finally linking both the fragments by the NCL method. The present inventors have further found that the thus-obtained full-length extracellular domain of AILIM containing an oligosaccharide chain added to Asn at residue 89 has approximately 10 times higher ability to bind to its ligand than that of a conventionally known soluble extracellular domain of AILIM, i.e., a fusion protein of the extracellular domain of AILIM with IgG Fc. Based on these findings, the present invention has been completed.
Specifically, the present invention relates to:

[1] an oligosaccharide chain-added extracellular domain of AILIM, comprising an extracellular domain of AILIM containing an oligosaccharide chain bound to a position corresponding to position 69 in the amino acid sequence of the extracellular domain of human AILIM represented by SEQ ID NO: 7, and no oligosaccharide chain added to a position corresponding to position 3 or 90 therein;
[2] an oligosaccharide chain-added extracellular domain of AILIM, comprising an extracellular domain of AILIM containing an oligosaccharide chain bound only to a position corresponding to position 69 in the amino acid sequence of the extracellular domain of human AILIM represented by SEQ ID NO: 7;
[3] the oligosaccharide chain-added extracellular domain of AILIM according to [1] or [2], wherein the oligosaccharide chain is an oligosaccharide chain represented by

[Formula 1]

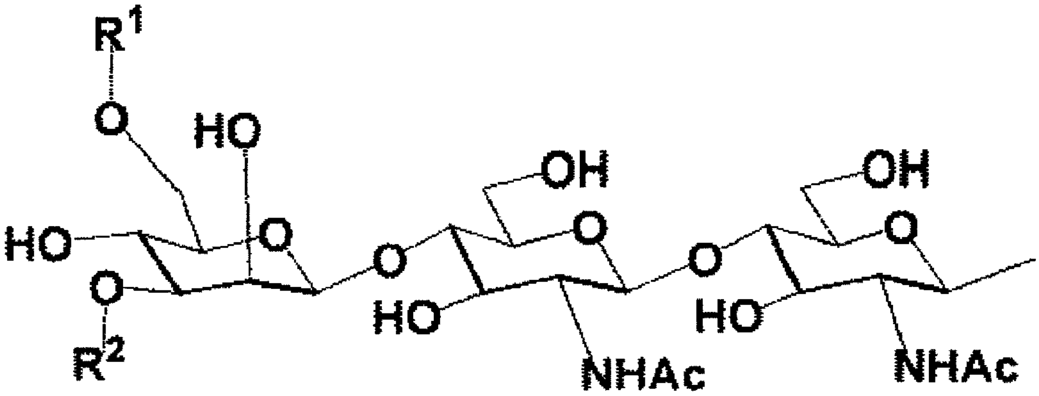

wherein $R^1$ and $R^2$, which are the same or different, each represent

[Formula 2]

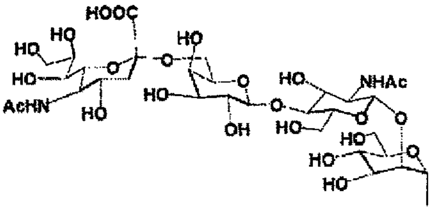,

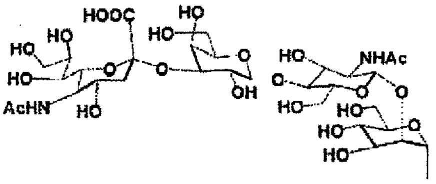,

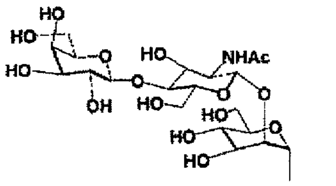,

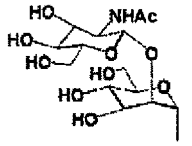,

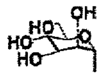 or

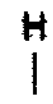

and Ac represents an acetyl group;

[4] an oligosaccharide chain-added extracellular domain of AILIM, comprising an extracellular domain of AILIM consisting of the amino acid sequence represented by SEQ ID NO: 7, the extracellular domain containing the following asialo-nonasaccharide chain bound only to Asn 69 of SEQ ID NO: 7:

[Formula 3]

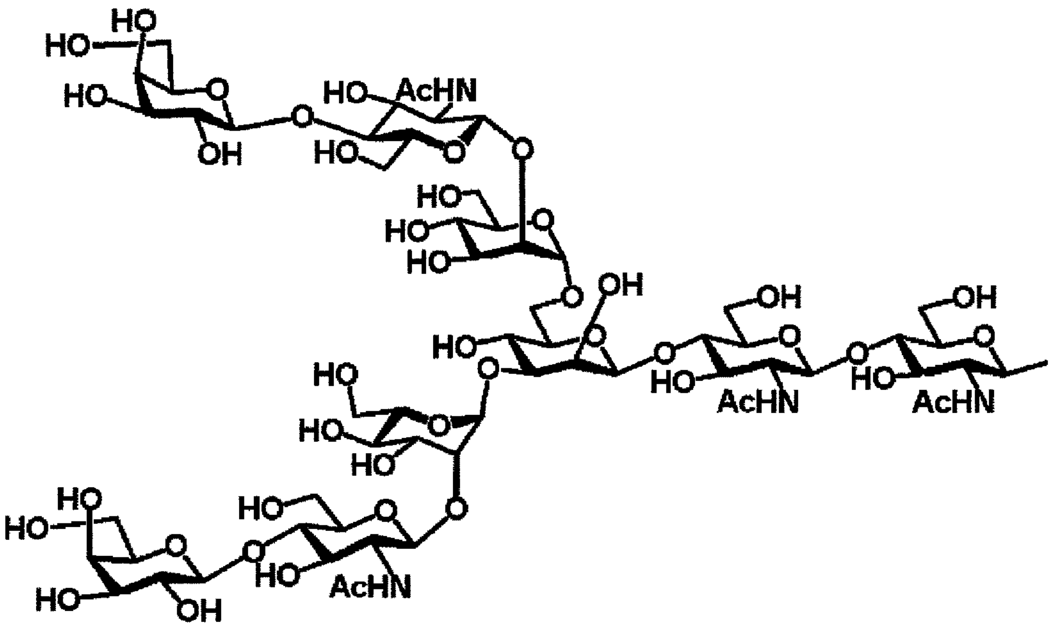

[5] the oligosaccharide chain-added extracellular domain according to any one of [1] to [4], wherein the oligosaccharide chain is substantially homogeneous;
[6] a method for producing an oligosaccharide chain-added extracellular domain of AILIM, comprising the steps of:

dividing the oligosaccharide chain-added extracellular domain of AILIM into the fragment A corresponding to a region of positions 1 to 42 in the amino acid sequence of the extracellular domain of human AILIM represented by SEQ ID NO: 7, the fragment B corresponding to a region of positions 43 to 62 in the amino acid sequence of the extracellular domain of human AILIM represented by SEQ ID NO: 7, the fragment C corresponding to a region of positions 63 to 88 and containing an oligosaccharide chain-added position corresponding to position 69 in the amino acid sequence of the extracellular domain of human AILIM represented by SEQ ID NO: 7, and the fragment D corresponding to a region of position 89 to the C-terminus in the amino acid sequence of the extracellular domain of human AILIM represented by SEQ ID NO: 7; and
preparing the fragments A to D, comprising:

introducing a protective group to the thiol group of Cys corresponding to position 22 in the fragment A; adding an oligosaccharide chain to Asn corresponding to position 69 in the fragment C; and introducing a protective group to the respective thiol groups of Cys 116 and Cys 117 in the fragment D;

thioesterifying the respective C-termini of the fragments A to C;
linking the fragments A and B by a KCL method;
linking the fragments C and D by an NCL method; and
linking the fragments A and B thus linked and the fragments C and D thus linked by the NCL method;

[7] the method according to [6], wherein the preparation of the fragment A is performed by a solid-phase synthesis method, wherein in the solid-phase synthesis method, Cys having a protective group introduced to the thiol group is used as the Cys corresponding to position 22 in the amino acid sequence of the extracellular domain of human AILIM represented by SEQ ID NO: 7;
[8] the method according to [6], wherein the preparation of the fragment C is performed by a solid-phase synthesis method, wherein in the solid-phase synthesis method, oligosaccharide chain-added asparagine is used as the Asn corresponding to position 69 in the amino acid sequence of the extracellular domain of human AILIM represented by SEQ ID NO: 7;
[9] the method according to [6], wherein the preparation of the fragment D is performed by a solid-phase synthesis method, wherein in the solid-phase synthesis method, Cys having a protective group introduced to the thiol group is used as each of the Cys 116 and the Cys 117 in the amino acid sequence of the extracellular domain of human AILIM represented by SEQ ID NO: 7;
[10] the method according to any one of [7] to [9], wherein the solid-phase synthesis method is an Fmoc solid-phase synthesis method;
[11] the method according to [7], wherein the Fmoc solid-phase synthesis method comprises microwave irradiation in amino acid condensation reaction and/or Fmoc group elimination reaction;
[12] the method according to any one of [6] to [11], wherein the oligosaccharide chain is an oligosaccharide chain represented by

[Formula 4]

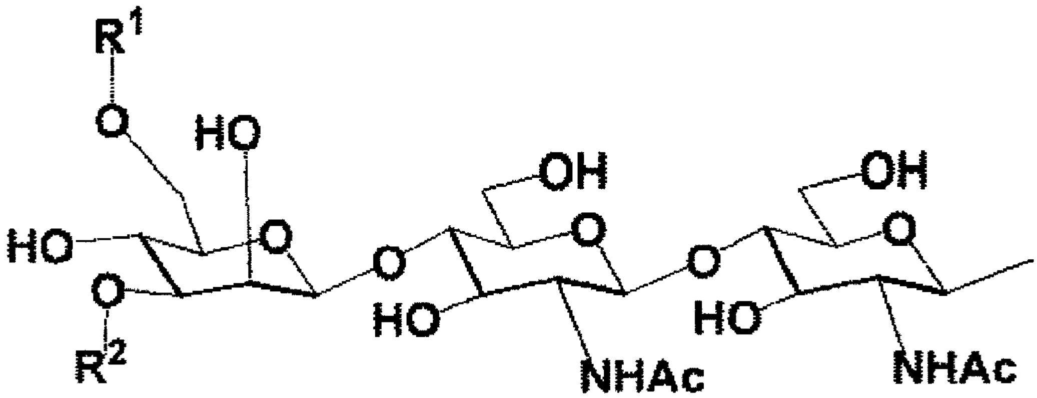

wherein $R^1$ and $R^2$, which are the same or different, each represent

[Formula 5]

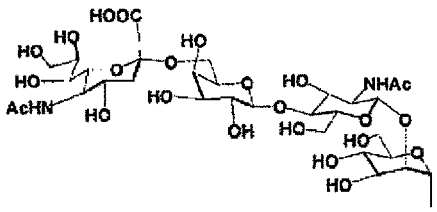
,
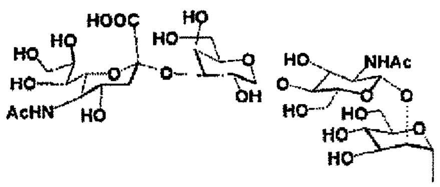
,
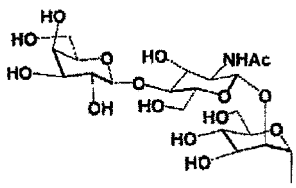
,
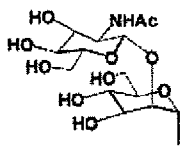
,
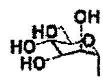
or
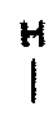

and Ac represents an acetyl group;

[13] the method according to [12], wherein the oligosaccharide chain is the following asialo-nonasaccharide chain:

[Formula 6]

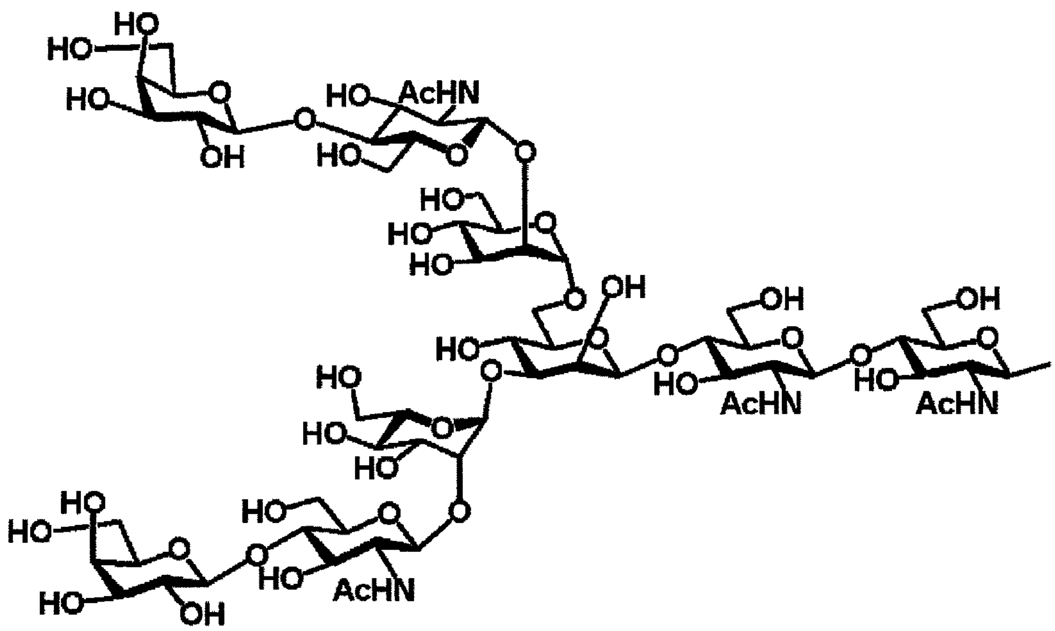

[14] an oligosaccharide chain-added extracellular domain of AILIM produced by a method according to any one of [6] to [13], wherein the oligosaccharide chain is substantially homogeneous;
[15] a pharmaceutical composition comprising an oligosaccharide chain-added extracellular domain of AILIM according to any one of [1] to [5] and [14] as an active ingredient;
[16] the pharmaceutical composition according to [15], wherein the pharmaceutical composition is intended for the treatment or prevention of an AILIM-related disease;
[17] the pharmaceutical composition according to [15], wherein the pharmaceutical composition is an immunosuppressive agent; and
[18] an immunosuppression method comprising administering an oligosaccharide chain-added extracellular domain of AILIM according to any one of [1] to [5] or a pharmaceutical composition according to any one of [15] to [17].

Advantageous Effects of Invention

An oligosaccharide chain-added extracellular domain of AILIM according to the present invention is a novel compound comprising an extracellular domain containing a oligosaccharide chain added to Asn at residue 89 and has approximately 10 times higher ability to bind to its ligand than a conventional product. The extracellular domain of AILIM is soluble and expected to be useful as pharmaceuticals.
Moreover, according to a production method according to the present invention, the formation of by-products can be inhibited, and a ligation method can be performed efficiently. Thus, an oligosaccharide chain-added extracellular domain of AILIM that has high quality available as pharmaceuticals can be produced in large amounts. Particularly, oligosaccharide chain-added fragments containing homogeneous oligosaccharide chains can be used as oligosaccharide chain-added fragments in ligation to thereby synthesize large amounts of oligosaccharide chain-added extracellular domains of AILIM having homogeneous oligosaccharide chain structures and glycosylation positions.

Brief Description of Drawings

[Figure 1] Figure 1 shows the comparison among the amino acid sequences of AILIMs (human, mouse, and rat), human CD28, and human CTLA4.

[Figure 2] Figure 2 shows a synthesis scheme based on a method for producing an oligosaccharide chain-added extracellular domain of AILIM of the present invention.

[Figure 3] Figure 3 shows results of analyzing the binding reactivity between the oligosaccharide chain-added extracellular domain of AILIM of the present invention and a ligand (B7h).

[Figure 4] Figure 4 shows results of analyzing the reactivity between the oligosaccharide chain-added extracellular domain of AILIM of the present invention and an anti-ICOS monoclonal antibody.

[Figure 5] Figure 5 shows one example of a previously studied synthesis scheme (conventional technique) of the extracellular domain of AILIM using a ligation method. In Figures 5 to 8, the numeric number in the parentheses is based on the conventional counting of amino acids in AILIM protein (counting with N-terminal Met as the first residue) .

[Figure 6] Figure 6 shows one example of a previously studied synthesis scheme (conventional technique) of the extracellular domain of AILIM using a ligation method.

[Figure 7] Figure 7 shows one example of a previously studied synthesis scheme (conventional technique) of the extracellular domain of AILIM using a ligation method.

[Figure 8] Figure 8 shows one example of a previously studied synthesis scheme (conventional technique) of the extracellular domain of AILIM using a ligation method.

Description of Embodiments

Hereinafter, preferable embodiments of the present invention will be described.

<Oligosaccharide chain-added extracellular domain of AILIM>

In the present invention, AILIM refers to a costimulatory receptor also called AILIM/ICOS. The AILIM according to the present invention encompasses those derived from arbitrary mammals such as humans, monkeys, mice, dogs, cattle, and horses. Human-derived AILIM is preferable for use as pharmaceuticals. It is known that the amino acid sequence of AILIM is well conserved between species with homology of 68% between human and mouse AILIMs and 84% between mouse and rat AILIMs. The amino acid sequence of human AILIM is shown in SEQ ID NO: 1. Moreover, the comparison among the respective amino acid sequences of rat, mouse, and human AILIMs is shown in Figure 1.

In the present specification, the term "residue X" represents the position of the residue that follows the conventional counting shown in Figure 1, i.e., counting with the N-terminal Met of AILIM as the first residue, unless otherwise specified. In this case, the extracellular domain of human AILIM is located at residues 21 to 141.

In the present invention, the extracellular domain of AILIM refers to a portion exposed at cell surface, except for the signal sequence, in AILIM expressed as a type I membrane protein. AILIM has the signal sequence (20 amino acid residues) at the N-terminus. A 121-amino acid residue (human) or 122-amino acid residue (rat or mouse) region subsequent thereto is typically the extracellular domain. The amino acid sequence of the extracellular domain of human AILIM is shown in SEQ ID NO: 7 as an example of the extracellular domain of AILIM.

Moreover, the extracellular domain of AILIM according to the present invention encompasses a peptide consisting of a region usually called the extracellular domain as well as a peptide comprising an amino acid sequence derived from the extracellular domain of AILIM by the deletion, substitution, or addition of one or more amino acid(s), a peptide comprising an amino acid sequence derived from the extracellular domain of AILIM by the conservative substitution of one or more amino acid(s), or a modified extracellular domain of AILIM, as long as the effect of the present invention is exerted. Thus, the extracellular domain of AILIM according to the present invention encompasses, for example, a 120-amino acid peptide (SEQ ID NO: 8) consisting of the amino acid sequence represented by SEQ ID NO: 7 that lacks Phe 121.

In the present specification, the term "amino acid" is used in the broadest sense and encompasses not only natural amino acids but nonnatural amino acids such as amino acid variants and derivatives. Those skilled in the art would understand that in consideration of this wide definition, examples of the amino acid according to the present specification include: natural proteinous L-amino acids; D-amino acids; chemically modified amino acids such as amino acid variants and derivatives; natural non-proteinous amino acids such as norleucine, β-alanine, and ornithine; and chemically synthesized compounds having properties known in the art as the features of amino acids. Examples of the nonnatural amino acids include α-methylamino acids (α-methylalanine, etc.), D-amino acids, histidine-like amino acids (2-amino-histidine, β-hydroxy-histidine, homohistidine, α-fluoromethyl-histidine, and α-methyl-histidine, etc.), amino acids having extra methylene in the side chain ("homo" amino acids), and amino acids comprising a sulfonic acid group substituted for a carboxylic acid functional group amino acid in the side chain (cysteic acid, etc.). In a preferable aspect, the amino acids contained in the compound of the present invention consist only of natural amino acids.

In the phrase "deletion, substitution, or addition of one or more amino acid(s)" in the present specification, the number of the substituted amino acid(s) or the like is not particularly limited as long as the resulting glycosylated extracellular domain of AILIM has the ability to bind to the AILIM ligand. The number is on the order of 1 to 9, preferably 1 to 5, more preferably 1 to 3 or within 10%, preferably 5%, of the whole length. Amino acid(s) substituted therefor or added thereto can be a natural amino acid, a nonnatural amino acid, or an amino acid analog and is preferably a natural amino acid. The deletion, substitution, and addition may be performed at any position in the amino acid sequence as long as these conditions are satisfied.
In this context, the AILIM ligand refers to a molecule that is expressed on antigen-presenting cells and binds to AILIM. Examples thereof include B7h (see e.g., Non Patent Literature 7), B7RP-1 (see e.g., Non Patent Literature 6), and GL50 (see e.g., Non Patent Literature 8).

In the present specification, the phrase "conservative substitution of one or more amino acid(s)" refers to amino acid substitution that occurs between the original amino acid and an amino acid to be substituted therefor having similar hydrophilicity indexes and/or hydrophobicity indexes so that such substitution does not cause apparent reduction or disappearance of the ability of the oligosaccharide chain-added extracellular domain of AILIM to bind to its ligand.

In the present specification, the phrase "modified extracellular domain of AILIM" refers to a naturally or artificially modified compound of the extracellular domain of AILIM. Examples of such modification include alkylation, acylation (e.g., acetylation), amidation, carboxylation, esterification, disulfide bond formation, glycosylation, lipidation, phosphorylation, hydroxylation, and labeling component binding of one or more amino acid residue(s) in the extracellular domain of AILIM.

An oligosaccharide chain-added extracellular domain of AILIM (sugar chain-added extracellular domain of AILIM, glycosylated extracellular domain of AILIM) of the present invention comprises the extracellular domain of AILIM described above containing an oligosaccharide chain added thereto. More specifically, the oligosaccharide chain is bound to a position corresponding to position 69 in the amino acid sequence of the extracellular domain of human AILIM represented by SEQ ID NO: 7.
In this context, since the extracellular domain of AILIM of the present invention encompasses, as described above, the "extracellular domain of AILIM derived from a non-human species", the "peptide comprising an amino acid sequence derived from the extracellular domain of AILIM by the deletion, substitution, or addition of one or more amino acid(s)", the "peptide comprising an amino acid sequence derived from the extracellular domain of AILIM by the conservative substitution of one or more amino acid(s)", the "modified extracellular domain of AILIM", etc., the phrase "position corresponding to position 69" means a position, in such a peptide or modified form, corresponding to position 69 in the amino acid sequence of the extracellular domain of human AILIM represented by SEQ ID NO: 7. This position can be determined easily by those skilled in the art based on each surrounding amino acid sequence or the like.
For example, in the extracellular domain of rat or mouse AILIM, the position corresponding to position 69 in the extracellular domain of human AILIM is, as also understood from the table shown in Figure 1, position 89 in the amino acid sequence of rat AILIM-1 represented by SEQ ID NO: 2, position 89 in the amino acid sequence of rat AILIM-2 represented by SEQ ID NO: 3, and position 89 in the amino acid sequence of mouse AILIM represented by SEQ ID NO: 4.
Examples of the oligosaccharide chain-added extracellular domain of AILIM of the present invention include an oligosaccharide chain-added extracellular domain of AILIM, comprising a 120-amino acid peptide (SEQ ID NO: 8) consisting of the amino acid sequence represented by SEQ ID NO: 7 that lacks Phe 121 and containing an oligosaccharide chain bound only to position 69 (SEQ ID NO: 9).

The amino acid at the position corresponding to position 69 is not necessarily required to be Asn as long as it is located at the position corresponding to position 69 of SEQ ID NO: 7. Those having an amino acid other than Asn substituted for the amino acid at the position corresponding to position 69 according to a glycosylation method or the like are also encompassed in the oligosaccharide chain-added extracellular domain of AILIM of the present invention.
In the present specification, the phrase "position corresponding to position Y of SEQ ID NO: X" in the description below is interpreted according to the description about the "position corresponding to position 69 of SEQ ID NO: 7".

Moreover, the oligosaccharide chain-added extracellular domain of AILIM of the present invention is characterized by comprising no oligosaccharide chain added to a position corresponding to position 3 or 90 in the amino acid sequence of the extracellular domain of human AILIM represented by SEQ ID NO: 7.

Moreover, the oligosaccharide chain-added extracellular domain of AILIM of the present invention may comprise one or more oligosaccharide chain(s) added to a position other than the position corresponding to position 69 of SEQ ID NO: 7 (except for a position corresponding to position 3 or 90 in the amino acid sequence of the extracellular domain of human AILIM represented by SEQ ID NO: 7), as long as the effect of the present invention is exerted.

In the present specification, the term "oligosaccharide chain (sugar chain)" refers to a compound that is formed by the linkage of one or more unit sugar(s) (monosaccharide and/or its derivative). In the linkage of two or more unit sugars, these unit sugars are bound to each other by dehydration condensation via the glycoside bond. Examples of such oligosaccharide chains include, but not limited to, monosaccharides and polysaccharides contained *in vivo* (glucose, galactose, mannose, fucose, xylose, N-acetylglucosamine, N-acetylgalactosamine, sialic acid, and complexes and derivatives thereof) as well as a wide range of oligosaccharide chains, for example, decomposed polysaccharides, and oligosaccharide chains decomposed or induced from complex biomolecules such as glycoprotein, proteoglycan, glycosaminoglycan, and glycolipid. The oligosaccharide chain may be in a linear or branched form.

Moreover, in the present specification, the "oligosaccharide chain" also encompasses oligosaccharide chain derivatives. Examples of the oligosaccharide chain derivatives include, but not limited to, oligosaccharide chains whose constituent sugars are sugars having a carboxyl group (e.g., aldonic acid which is carboxylic acid formed by oxidation at C-1 (e.g., D-gluconic acid which is formed by the oxidation of D-glucose) and uronic acid which contains the terminal C atom converted to carboxylic acid (D-glucuronic acid which is formed by the oxidation of D-glucose)), sugars having an amino group or an amino group derivative (e.g., an acetylated amino group) (e.g., N-acetyl-D-glucosamine and N-acetyl-D-galactosamine), sugars having both amino and carboxyl groups (e.g., N-acetylneuraminic acid (sialic acid) and N-acetylmuramic acid), deoxy sugars (e.g., 2-deoxy-D-ribose), sulfated sugars containing a sulfuric acid group, or phosphorylated sugars containing a phosphoric acid group.

In the present invention, the oligosaccharide chain is preferably, for example, an oligosaccharide chain that increases the ability to bind to the ligand when added to the extracellular domain of AILIM.

The oligosaccharide chain in the oligosaccharide chain-added extracellular domain of AILIM of the present invention is an oligosaccharide chain that is present as a complex carbohydrate (glycopeptide (or glycoprotein), proteoglycan, glycolipid, etc.) *in vivo,* preferably, an oligosaccharide chain that is bound to a peptide (or protein) in a glycopeptide (or glycoprotein) *in vivo,* such as an N-linked oligosaccharide chain or an O-linked oligosaccharide chain, from the viewpoint of administering the oligosaccharide chain-added extracellular domain of AILIM of the present invention to an organism.

Among them, the N-linked oligosaccharide chain is preferable. Examples of the N-linked oligosaccharide chain can include a high-mannose type, a complex type, and a hybrid type. The complex type is particularly preferable.

In an aspect of the present invention, the oligosaccharide chain in the oligosaccharide chain-added extracellular domain of AILIM of the present invention is preferably an oligosaccharide chain consisting of 4 or more, for example, 5 or more or 7 or more, particularly, 9 or more sugars.

In a preferable aspect of the present invention, the oligosaccharide chain in the oligosaccharide chain-added extracellular domain of AILIM is an oligosaccharide chain consisting of 5 to 11, 9 to 11, or 9 sugars.

In a preferable aspect of the present invention, the oligosaccharide chain in the oligosaccharide chain-added extracellular domain of AILIM of the present invention is an oligosaccharide chain selected from the group consisting of a disialo oligosaccharide chain, a monosialo oligosaccharide chain, an asialo oligosaccharide chain, a diGlcNAc oligosaccharide chain, and dimannose oligosaccharide chain and is more preferably an asialo oligosaccharide chain.

Examples of the preferable oligosaccharide chain used in the present invention include oligosaccharide chains represented by the following general formula:

[Formula 7]

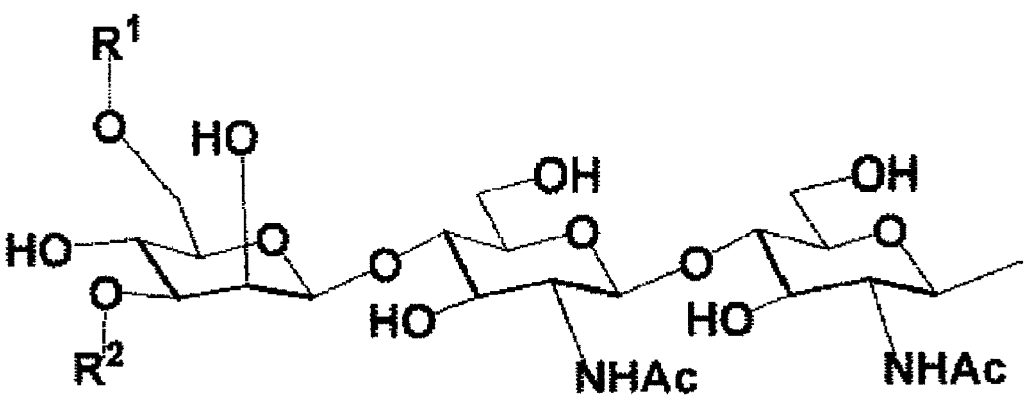

wherein $R^1$ and $R^2$, which are the same or different, each represent

[Formula 8]

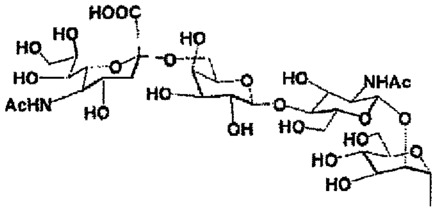
,
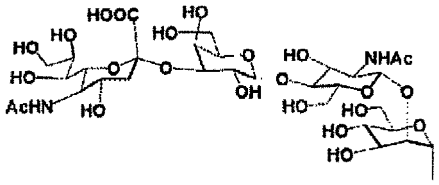
,
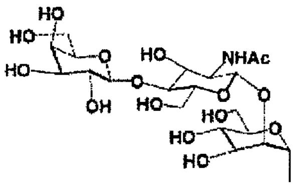
,
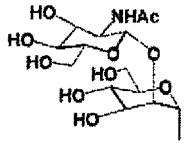
,
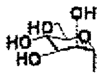
or
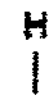

and Ac represents an acetyl group.

The added oligosaccharide chain in the extracellular domain of AILIM of the present invention is preferably an asialo-nonasaccharide chain shown below. Particularly, the oligosaccharide chain-added extracellular domain of AILIM of the present invention is preferably the 121-amino acid extracellular domain of AILIM represented by SEQ ID NO: 7 or a 120-amino acid extracellular domain of AILIM (SEQ ID NO: 8) containing the following asialo-nonasaccharide chain added to position 69:

[Formula 9]

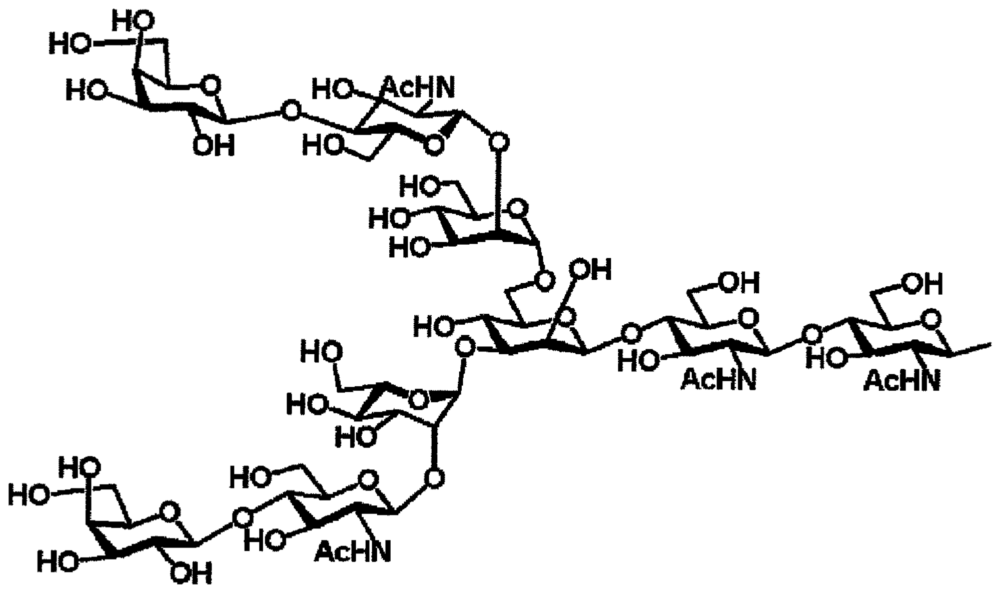

In the present invention, examples of other preferable oligosaccharide chains can include oligosaccharide chains shown below in Tables 1 to 4, such as oligosaccharide chains having the same structure (oligosaccharide chains having the same type of constituent sugars and the same binding pattern thereof) as that of a oligosaccharide chain that is present in human bodies as a glycoprotein comprising the oligosaccharide chain bound to a protein (e.g., an oligosaccharide chain described in "FEBS LETTERS Vol. 50, No. 3, Feb. 1975"), and oligosaccharide chains derived therefrom by the deletion of one or more sugar(s) from the non-reducing end.

[Table 1]

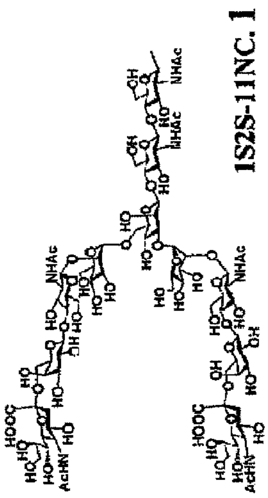

1S2S-11NC, 1

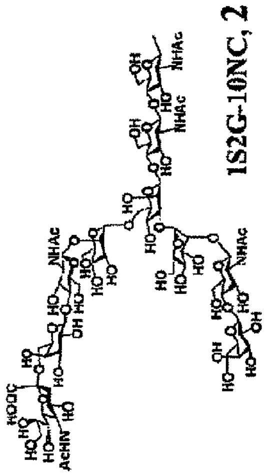

1S2G-10NC, 2

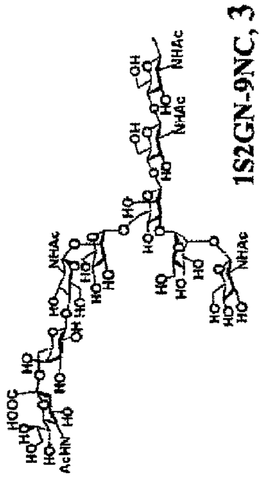

1S2GN-9NC, 3

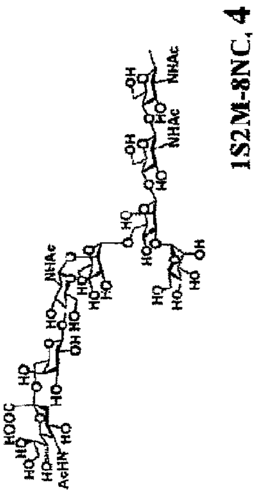

1S2M-8NC, 4

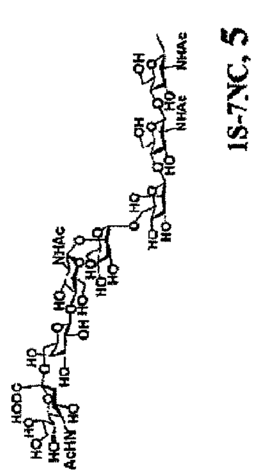

1S-7NC, 5

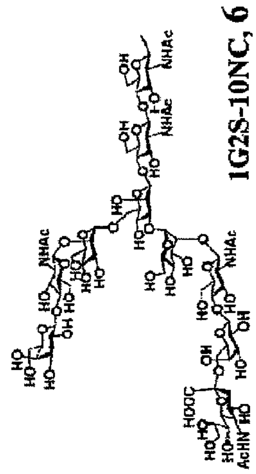

1G2S-10NC, 6

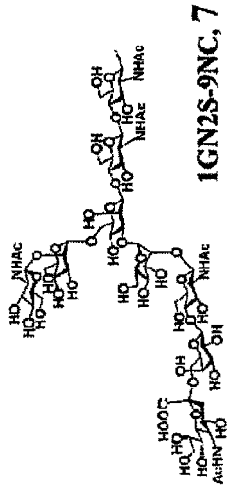

1GN2S-9NC, 7

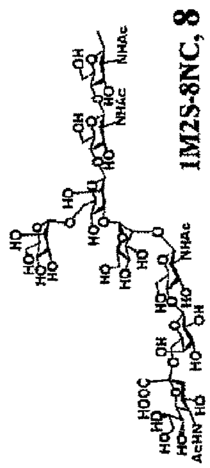

1M2S-8NC, 8

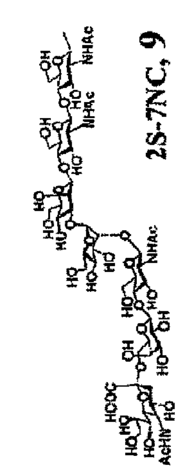

2S-7NC, 9

[Table 2]

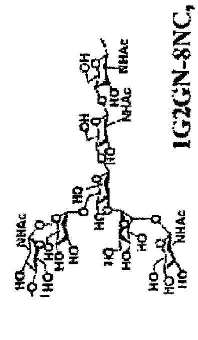
1G2GN-8NC, 10

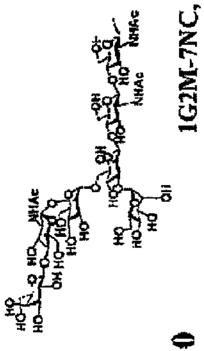
1G2M-7NC, 11

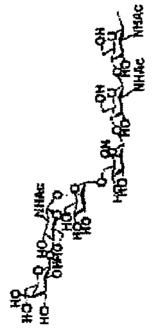
1G-6NC, 12

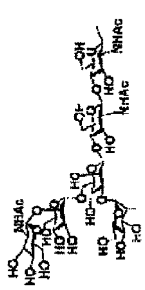
1GN2M-6NC, 13

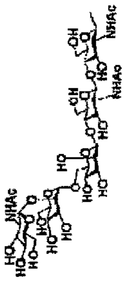
1GN-5NC, 14

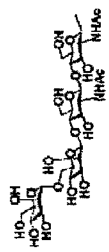
1M-4NC, 15

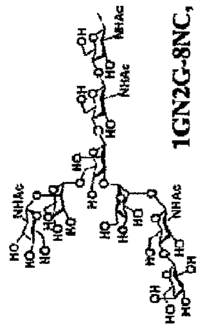
1GN2G-8NC, 16

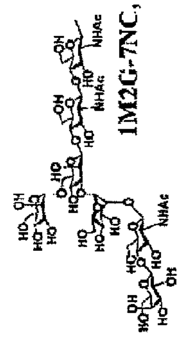
1M2G-7NC, 17

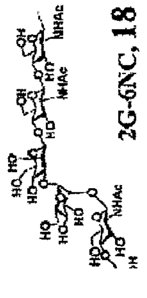
2G-6NC, 18

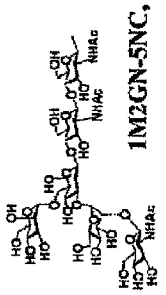
1M2GN-5NC, 19

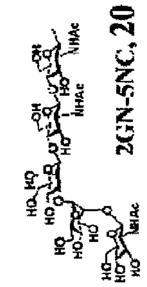
2GN-5NC, 20

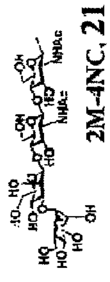
2M-4NC, 21

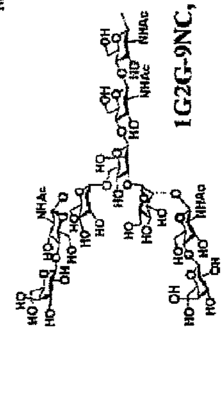
1G2G-9NC, 22

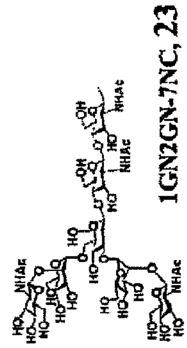
1GN2GN-7NC, 23

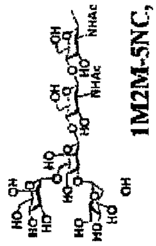
1M2M-5NC, 24

[Table 3]

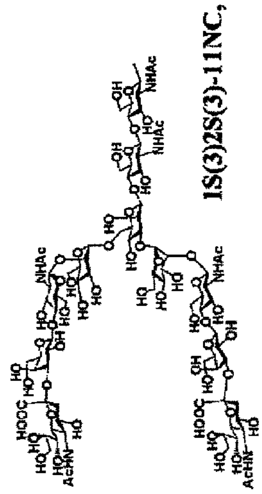
1S(3)2S(3)-11NC, 25

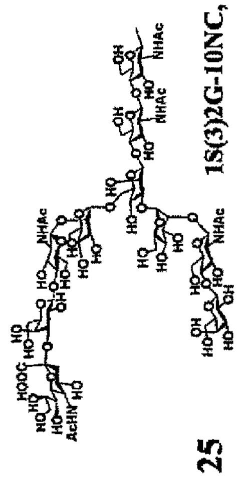
1S(3)2G-10NC, 26

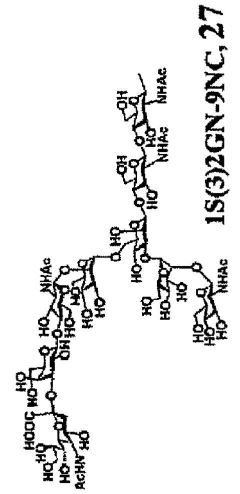
1S(3)2GN-9NC, 27

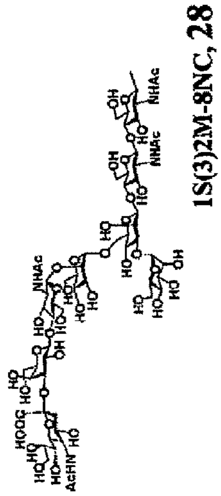
1S(3)2M-8NC, 28

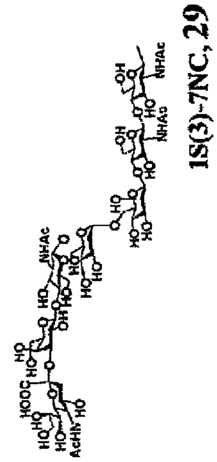
1S(3)-7NC, 29

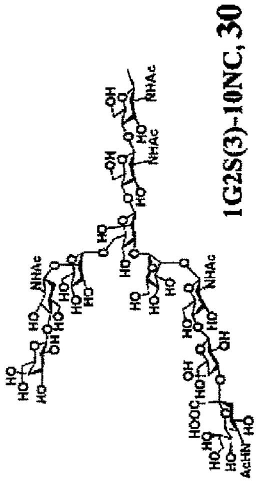
1G2S(3)-10NC, 30

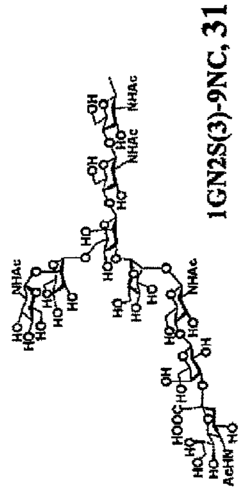
1GN2S(3)-9NC, 31

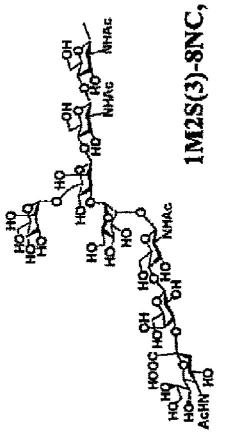
1M2S(3)-8NC, 32

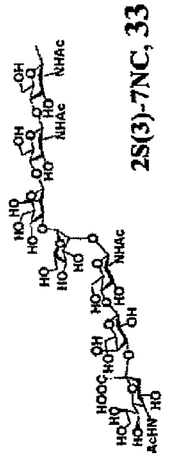
2S(3)-7NC, 33

[Table 4]

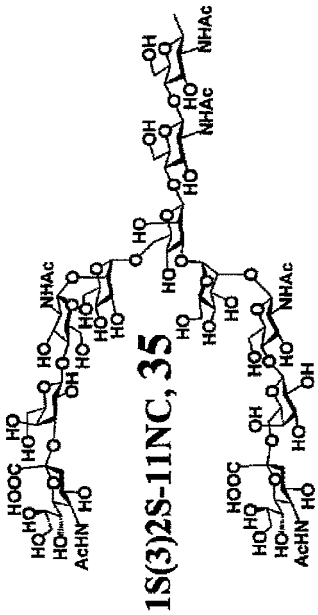

1S(3)2S-11NC, 35

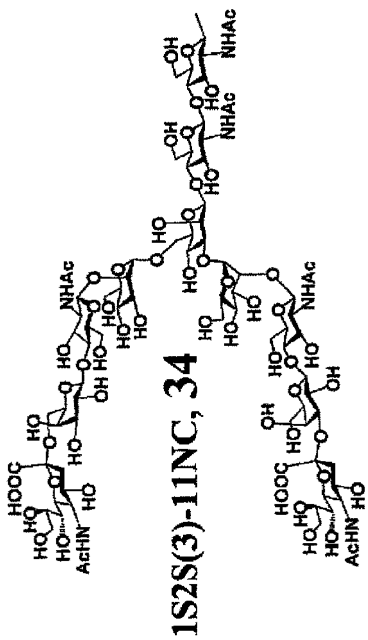

1S2S(3)-11NC, 34

In the present specification, the term "oligosaccharide chain-added amino acid (sugar chain added-amino acid,

glycosylated amino acid)" refers to an amino acid bound to an oligosaccharide chain. The amino acid may be bound to the oligosaccharide chain via a linker. The amino acid binding site in the oligosaccharide chain is not particularly limited, and it is preferred that the amino acid should be bound to the reducing end of the oligosaccharide chain.
The type of the amino acid bound to the oligosaccharide chain is not particularly limited and is any of the above-described amino acids in the extracellular domain of AILIM. Preferable examples thereof include Asn, Ser, Cys, and Lys. Asn is more preferable.

When the amino acid is bound to the oligosaccharide chain via a linker, examples of the type of the linker can include, but not particularly limited to, $-NH-(CO)-(CH_2)_a-CH_2-$ (wherein a represents an integer, preferably an integer of 0 to 4, but not limited thereto unless the linker function of interest is inhibited), $C_{1-10}$ polymethylene, and $-CH_2-R-$ (wherein R represents a group that is formed by the elimination of one hydrogen atom from a group selected from the group consisting of alkyl, substituted alkyl, alkenyl, substituted alkenyl, alkynyl, substituted alkynyl, aryl, substituted aryl, a carbocyclic group, a substituted carbocyclic group, a heterocyclic group, and a substituted heterocyclic group).

The oligosaccharide chain-added extracellular domain of AILIM according to the present invention may be produced by any production method and may be produced, for example, by a solid-phase synthesis method using an oligosaccharide chain-added amino acid as a starting material or by binding an amino acid-unbound oligosaccharide chain to an amino acid in the peptide either directly or via a linker. The oligosaccharide chain-unbound peptide can be prepared by solid-phase synthesis, liquid-phase synthesis, expression using an expression system, or the like.
Moreover, the peptide is divided into fragments, which may each be synthesized and linked according to a ligation method, as in a method for producing the oligosaccharide chain-added extracellular domain of AILIM of the present invention, which will be described later.
Moreover, the oligosaccharide chain-added extracellular domain of AILIM of the present invention also encompasses, for example, those produced by oligosaccharide chain elongation by further adding a sugar or a sugar chain to the oligosaccharide chain in the oligosaccharide chain-added extracellular domain of AILIM, as long as they have a final structure identical to that of the present invention.

In a preferable aspect of the present invention, the oligosaccharide chain added to the extracellular domain of AILIM has a substantially homogeneous structure. In the present specification, the phrase "oligosaccharide chain has a substantially homogeneous structure" means that a glycosylation site, the types of the constituent sugars in the oligosaccharide chain, the order of binding, and the binding pattern between the sugars are the same when compared among oligosaccharide chain-added extracellular domains of AILIM, and means that the oligosaccharide chain has a structure with at least 90% or more, preferably 95% or more, more preferably 99% or more homogeneity. Glycopeptides having homogeneous oligosaccharide chains have constant quality and are particularly preferable for fields such as pharmaceutical production and assay. The contents of homogeneous oligosaccharide chains can be measured by a method using, for example, HPLC, capillary electrophoresis, NMR, or mass spectrometry. The production of the oligosaccharide chains whose structures are homogeneous is described in the pamphlet of International Publication No. WO 03/008431 (US2004181054 (A1)), the pamphlet of International Publication No. WO 2004/058984 (US2006228784 (A1)), the pamphlet of International Publication No. WO 2004/058824 (US2006009421 (A1)), the pamphlet of International Publication No. WO 2004/070046 (US2006205039 (A1)), and the pamphlet of International Publication No. WO 2007/011055. The disclosure thereof is incorporated herein by reference in its entirety.

The oligosaccharide chain-added extracellular domain of AILIM of the present invention has approximately 10 times or higher ability to bind to the ligand than that of a conventionally known soluble extracellular domain of AILIM, i.e., a fusion protein of the extracellular domain of AILIM with IgG Fc. As described later, the oligosaccharide chain-added extracellular domain of AILIM of the present invention does not react with an anti-ICOS monoclonal antibody. This suggests that an epitope recognized by the monoclonal antibody is not formed in the oligosaccharide chain-added extracellular domain of AILIM of the present invention. It has not been expected at all that the soluble extracellular domain of AILIM thus differing in both the number of oligosaccharide chains and higher-order structure from wild-type AILIM would have the exceedingly high ability to bind to the ligand. It has previously been confirmed that the AILIM-IgG Fc fusion protein exhibits immunosuppressive effect in heart transplantation (see e.g., Test 2 in Japanese Patent Laid-Open No. 2003-277293). Thus, the oligosaccharide chain-added extracellular domain of AILIM of the present invention is understood to be highly useful as immunosuppressive agents or preventive or therapeutic agents for an AILIM-related disease.

<Method for producing oligosaccharide chain-added extracellular domain of AILIM>

Next, a method for producing the oligosaccharide chain-added extracellular domain of AILIM according to the present invention will be described.

The method for producing the oligosaccharide chain-added extracellular domain of AILIM according to the present invention comprises the steps of: dividing the oligosaccharide chain-added extracellular domain of AILIM to four fragments (A to D) and preparing each of these fragments; and sequentially linking the fragments by a KCL method or

an NCL method.

The four fragments are a fragment A corresponding to a region of positions 1 to 42 in the amino acid sequence of the extracellular domain of human AILIM represented by SEQ ID NO: 7, a fragment B corresponding to positions 43 to 62, a fragment C corresponding to positions 63 to 88, and a fragment D corresponding to position 89 to the C-terminus.

A method for preparing each fragment is not particularly limited, and each fragment can be prepared by a peptide synthesis method known in the art, such as a solid-phase synthesis method, a liquid-phase synthesis method, or expression using an expression system, or a method equivalent thereto. The solid-phase synthesis method is preferable because the amino acid sequence can be elongated accurately. A glycosylation position may be controlled accurately by performing solid-phase synthesis using an oligosaccharide chain-added amino acid. Moreover, a protective group can also be introduced selectively to an amino acid residue susceptible to side reaction. On the other hand, it is also preferred that a long fragment should be produced efficiently using an expression system. Those skilled in the art can appropriately select the synthesis method depending on the amino acid sequences and lengths of fragments to be produced, the presence or absence of an oligosaccharide chain, etc. Hereinafter, the preparation of the fragments by the solid-phase synthesis method will be described in more detail.

(Solid-phase synthesis of fragments)

The solid-phase synthesis of the fragments can be performed according to a method known in the art or a method equivalent thereto.

For example, a fragment to which an oligosaccharide chain is not added can be synthesized as an arbitrary amino acid sequence by the following steps (1) to (4):

(1) Esterification reaction is performed between the hydroxy group of a hydroxy group-containing resin and the carboxyl group of an amino acid having amino group nitrogen protected with a fat-soluble protective group. In this case, since the amino group nitrogen in the amino acid is protected with a fat-soluble protective group, self-condensation between amino acids can be prevented. Thus, the hydroxy group of the resin is reacted with the carboxyl group of the amino acid to cause esterification.

(2) The fat-soluble protective group in the ester thus obtained is eliminated to form a free amino group.

(3) Steps (i) and (ii) shown below are performed once or more. As a result, arbitrary numbers of arbitrary amino acids are linked to obtain a peptide having the resin bound to the C-terminus and a free amino group at the N-terminus.

(i) Amidation reaction is performed between this free amino group and the carboxyl group of an arbitrary amino acid having amino group nitrogen protected with a fat-soluble protective group.

(ii) The fat-soluble protective group is eliminated to form a free amino group.

(4) The resin is cleaved off with an acid.

Moreover, for an oligosaccharide chain-added fragment, for example, a method described in the pamphlet of International Publication No. WO 2004/005330 (US2005222382 (A1)) can be used. The disclosure thereof is incorporated herein by reference in its entirety.

Specifically, amino acids are synthesized from the C-terminus to an amino acid just before the oligosaccharide chain-added amino acid by the steps (1) to (3). Then, amidation reaction is performed between the carboxyl group in the asparagine moiety of oligosaccharide chain-added asparagine having amino group nitrogen protected with a fat-soluble protective group and the free amino group formed in the step (3). Next, the fat-soluble protective group in the oligosaccharide chain-added asparagine is eliminated to form a free amino group.

Then, the step (3) is repeated necessary times. Finally, the resin can be cleaved off with an acid to obtain an oligosaccharide chain-added peptide fragment comprising an arbitrary amino acid sequence containing an oligosaccharide chain added to an arbitrary position.

In the step (1), esterification reaction can be performed between the hydroxy group of a hydroxy group-containing resin and the carboxyl group of an oligosaccharide chain-added amino acid having amino group nitrogen protected with a fat-soluble protective group to obtain an oligosaccharide chain-added peptide fragment containing an oligosaccharide chain added to the C-terminal amino acid.

Moreover, the synthesis is terminated immediately after the binding of the oligosaccharide chain-added amino acid having amino group nitrogen protected with a fat-soluble protective group. The resin can be cleaved off with an acid to obtain an oligosaccharide chain-added peptide fragment containing an oligosaccharide chain added to the N-terminal amino acid.

The hydroxy group-containing resin can be any hydroxy group-containing resin usually used in solid-phase synthesis. For example, Amino-PEGA resin (manufactured by Merck Ltd.), Wang resin (manufactured by Merck Ltd.),

and HMPA-PEGA resin (manufactured by Merck Ltd.) can be used. The HMPB-PEGA resin is preferable from the viewpoint of performing thioesterification after solid-phase synthesis.

Any amino acid can be used as the amino acids. Examples thereof can include natural amino acids such as serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophan (Trp), and proline (Pro).

Examples of the fat-soluble protective group can include carbonate- or amide-based protective groups such as 9-fluorenylmethoxycarbonyl (Fmoc), t-butyloxycarbonyl (Boc), benzyl, allyl, allyloxycarbonyl, and acetyl groups. For introducing the fat-soluble protective group, for example, the Fmoc group can be introduced by adding thereto 9-fluorenylmethyl-N-succinimidyl carbonate and sodium bicarbonate and performing reaction. It is preferred that the reaction should be performed at 0 to 50°C, preferably room temperature, for approximately 1 to 5 hours. A solid-phase synthesis method using the Fmoc group as a fat-soluble protective group is called an Fmoc solid-phase synthesis method.

The amino acid protected with a fat-soluble protective group can be produced by producing the amino acid by the method described above. Moreover, a commercially available product may be used. Examples thereof can include Fmoc-Ser, Fmoc-Asn, Fmoc-Val, Fmoc-Leu, Fmoc-Ile, Fmoc-Ala, Fmoc-Tyr, Fmoc-Gly, Fmoc-Lys, Fmoc-Arg, Fmoc-His, Fmoc-Asp, Fmoc-Glu, Fmoc-Gln, Fmoc-Thr, Fmoc-Cys, Fmoc-Met, Fmoc-Phe, Fmoc-Trp, Fmoc-Pro.

For example, a dehydration-condensation agent known in the art, such as 1-mesitylenesulfonyl-3-nitro-1,2,4-triazole (MSNT), dicyclohexylcarbodiimide (DCC), or diisopropylcarbodiimide (DIPCDI) can be used as an esterification catalyst. The ratio between the amino acid and the dehydration-condensation agent used is usually 1 to 10 parts by weight, preferably 2 to 5 parts by weight of the latter, with respect to 1 part by weight of the former.

It is preferred that the esterification reaction should be performed, for example, by placing a resin in a solid-phase column, washing this resin with a solvent, and then adding thereto an amino acid solution. Examples of the solvent for washing can include dimethylformamide (DMF), 2-propanol, methylene chloride, and etc. Examples of a solvent used for dissolving the amino acid can include dimethyl sulfoxide (DMSO), DMF, and methylene chloride. It is preferred that the esterification reaction should be performed at 0 to 50°C, preferably room temperature, for approximately 10 minutes to 30 hours, preferably approximately 15 minutes to 24 hours.

In this case, it is also preferred that unreacted hydroxy groups on the solid phase should be capped by acetylation using acetic anhydride or the like.

The elimination of the fat-soluble protective group can be performed, for example, by treatment with a base. Examples of the base can include piperidine morpholine, and etc. It is preferred that this treatment should be performed in the presence of a solvent. Examples of the solvent can include DMSO, DMF, methanol and etc.

It is preferred that the amidation reaction between the free amino group and the carboxyl group of an arbitrary amino acid having amino group nitrogen protected with a fat-soluble protective group should be performed in the presence of an activator and a solvent.

Examples of the activator can include dicyclohexylcarbodiimide (DCC), 1-ethyl-3-(3-dimethylaminopropyl)carbodiimide hydrochloride (WSC/HC1), diphenylphosphorylazide (DPPA), carbonyldiimidazole (CDI), diethyl cyanophosphonate (DEPC), diisopropylcarbodiimide (DIPCI), benzotriazol-1-yloxy-tris-pyrrolidino-phosphonium hexafluorophosphate (PyBOP), 1-hydroxybenzotriazole (HOBt), hydroxysuccinimide (HOSu), dimethylaminopyridine (DMAP), 1-hydroxy-7-azabenzotriazole (HOAt), hydroxyphthalimide (HOPht), pentafluorophenol (Pfp-OH), 2-(1H-benzotriazol-1-yl)-1,1,3,3-tetramethyluronium hexafluorophosphate (HBTU), O-(7-azabenzotriazol-1-yl)-1,1,3,3-tetramethyluronium hexafluorophosphonate (HATU), O-benzotriazol-1-yl-1,1,3,3-tetramethyluronium tetrafluoroborate (TBTU), and 3,4-dihydro-3-hydrodi-4-oxa-1,2,3-benzotriazine (Dhbt) and etc.

It is preferred that the amount of the activator used should be set to 1 to 20 equivalents, preferably 1 to 10 equivalents, more preferably 1 to 5 equivalents, with respect to the arbitrary amino acid having amino group nitrogen protected with a fat-soluble protective group.

Examples of the solvent can include DMSO, DMF, and methylene chloride. It is preferred that the reaction should be performed at 0 to 50°C, preferably room temperature, for approximately 10 to 30 hours, preferably approximately 15 minutes to 24 hours. The fat-soluble protective group can be eliminated in the same way as above.

Treatment with an acid is preferable for cleaving the peptide chain from the resin. Examples of the acid can include trifluoroacetic acid (TFA), hydrogen fluoride (HF) and etc.

Moreover, in the present invention, it may be preferred that a microwave method should be used in the solid-phase synthesis method. The microwave method is described in, for example, Bacsa B. et al., J. Org. Chem. (2008) 73: 7532-7542. The disclosure thereof is incorporated herein by reference in its entirety.

The microwave method is a method by which problems based on intramolecular aggregation, formation of secondary structures, steric hindrance caused by protective groups, etc., are solved by microwave irradiation in an amino acid condensation step or a deprotection step. This method is useful for synthesizing a peptide that is difficult to synthesize by a usual method, particularly, a long-chain peptide. Those skilled in the art can appropriately determine the microwave irradiation conditions, depending on the amino acid sequence, to prevent side reaction from occurring due to application

of heat or energy during the reaction.

For the method for producing the oligosaccharide chain-added extracellular domain of AILIM according to the present invention, it is preferred that the fragments A, C, and D should be prepared by the solid-phase synthesis method. On the other hand, the preparation of the fragment B may be performed by any of methods such as solid-phase synthesis, liquid-phase synthesis, expression using an expression system and etc.

Hereinafter, preferable preparation methods for the fragments A, C, and D will be described.

The fragment A corresponds to a fragment that has conventionally been obtained by the present inventors by synthesizing each of a fragment 1 (residues 21 to 41) and a fragment 2 (residues 42 to 62) and linking them by the KCL method. For the method according to the present invention, it is preferred that this fragment should be synthesized continuously by the solid-phase synthesis method. In the solid-phase synthesis of the fragment A, Cys containing amino group nitrogen protected with a fat-soluble protective group and a protective group introduced in the thiol group is used for binding Cys corresponding to position 22 of SEQ ID NO: 7. In this way, the fragment A can be synthesized continuously by the solid-phase synthesis method to thereby selectively protect Cys and prevent by-products from being formed due to the reaction of the thiol group in the Cys residue with a thioester during subsequent ligation by the NCL method or the KCL method.

Examples of the protective group introduced in the thiol group include, but not limited to, disulfide, acetamidomethyl (Acm), nitrobenzyl, and trityl groups.

Since the fragment A is relatively long, it is preferred to use the microwave method described above. Preferable irradiation conditions for amino acid condensation by solid-phase synthesis involve, for example, approximately 30°C to 50°C, preferably approximately 35°C to 40°C, approximately 10 minutes to 20 minutes, preferably approximately 15 minutes, and approximately 10 W to 30 W, preferably approximately 20 W. Preferable irradiation conditions for protective group removal involve, for example, approximately 30°C to 45°C, preferably approximately 35°C to 37°C, approximately 1 minute to 10 minutes, preferably approximately 3 to 5 minutes, approximately 10 W to 30 W, preferably approximately 20 W.

The fragment C can be synthesized according to the solid-phase synthesis method for the oligosaccharide chain-added peptide fragment described above.

Oligosaccharide chain-added Asn containing a substantially homogeneous oligosaccharide chain, used in the solid-phase synthesis of the oligosaccharide chain-added peptide fragment is described in, for example, the pamphlet of International Publication No. WO 03/008431 (US2004181054 (A1)), the pamphlet of International Publication No. WO 2004/058984 (US2006228784 (A1)), the pamphlet of International Publication No. WO 2004/058824 (US2006009421 (A1)), the pamphlet of International Publication No. WO 2004/070046 (US2006205039 (A1)), and the pamphlet of International Publication No. WO 2007/011055, as described above. The disclosure thereof is incorporated herein by reference in its entirety.

In the solid-phase synthesis of the fragment D, Cys containing amino group nitrogen protected with a fat-soluble protective group and a protective group introduced in the thiol group is used for binding Cys corresponding to each of positions 116 and 117 of SEQ ID NO: 7. In this way, Cys can be protected selectively. Thus, by-products can be prevented from being formed due to the reaction of the thiol group in the side chain of the Cys residue with a thioester during subsequent ligation by the NCL method or the KCL method. The same protective group as that for the fragment A can be used as the protective group introduced in the thiol group.

As a matter of course, the fragments A to D may be synthesized sequentially or concurrently. Moreover, when these fragments are synthesized sequentially, the order of synthesis is not limited.

(Step of thioesterifying C-terminus)

In the method for producing the oligosaccharide chain-added extracellular domain of AILIM according to the present invention, the respective C-termini of the fragments A to C are thioesterified, i.e., an α-carboxythioester moiety is formed at the C-termini, for performing ligation. A method known in the art or a method equivalent thereto can be used as a method for producing a peptide fragment (or glycopeptide fragment) having an α-carboxythioester moiety represented by - C(=O)-SR at the C-terminus. Such a method is described in, for example, the pamphlet of International Publication No. WO 96/34878 (US Patent No. 6184344). The disclosure thereof is incorporated herein by reference in its entirety.

In this context, R is not particularly limited as long as it is a group that does not inhibit thiol exchange reaction and becomes a leaving group in the nucleophilic substitution reaction of carbonyl carbon. Preferably, R can be selected from, for example, a benzyl form such as benzylmercaptan, an aryl form such as thiophenol or 4-(carboxymethyl)-thiophenol, an alkyl form such as 2-mercaptoethanesulfonate or 3-mercaptopropionic acid amide, and etc.

Specific examples of the method for producing a peptide fragment having an α-carboxythioester moiety at the C-terminus include a method by which thioesterification is performed during peptide excision from the solid phase, and a method by which the C-terminal carboxyl group of the peptide is thioesterified after peptide excision from the solid

phase. For example, a method comprising producing a peptide on a solid-phase resin using Safety Catch Linker and allowing a thiol compound to act thereon is known (J. Am. Chem. Soc., (1999) 121: 11369-11374; and Angew. Chem. Int. Ed., (2005) 44: 1650-1654) as the method by which thioesterification is performed during peptide excision from the solid phase. Moreover, when the C-terminal carboxyl group of the peptide is thioesterified after peptide excision from the solid phase, a peptide containing protected side chains can be reacted with alkylthiol or thiophenol.

For example, as described later in Examples, a protected peptide (or glycopeptide) containing protected amino acid side chains and N-terminal amino group is obtained by the solid-phase synthesis method, and its C-terminal carboxyl group is condensed with benzylthiol using PyBOP (benzotriazol-1-yl-oxy-tris-pyrrolidino-phosphonium hexafluorophosphate) /DIPEA as a condensing agent in a liquid phase. Then, the amino acid chain can be deprotected using a 95% TFA solution to obtain a peptide (or glycopeptide) thus having an $\alpha$-carboxythioester moiety at the C-terminus.

In the method for producing the oligosaccharide chain-added extracellular domain of AILIM according to the present invention, the step of preparing fragments A to D and the step of thioesterifying the respective C-termini of the fragments A to C may be performed simultaneously.

In a method for simultaneously performing the step of preparing fragments A to D and the step of thioesterifying the respective C-termini of the fragments A to C, for example, the amino acid sequence of intein is added to the respective C-termini of the fragments to prepare fusion proteins, and the intein is cleaved off by intein reaction while the C-termini of the fragments can be thioesterified. Such a method is described in, for example, Muralidharan V, et al., Nature Methods (2006) Vol. 3, No. 6, 429-438. The disclosure thereof is incorporated herein by reference in its entirety.

Since the fragments A and B are linked by the KCL method, the C-terminal thioester of the fragment A must be prepared as a thioester more highly eliminable than the C-terminal thioester of the fragment B. As a result, the fragment B can be linked to the C-terminus of the fragment A based on difference in reaction rate. Thus, the formation of by-products resulting from reverse linking, or the like, can be inhibited.

In general, thiol groups have stronger reactivity in the following order: an arylthiol group, a benzyl-type thiol group, and an alkyl-type thiol group. Based on this order, a thiol group having higher reactivity is prepared at the C-terminus of the fragment A than that at the C-terminus of the fragment B.

For example, a combination of a thiophenyl ester at the C-terminus of the fragment A and an ethyl thioester at the C-terminus of the fragment B, a combination of a 4-mercaptophenyl thioester (MPAA) at the C-terminus of the fragment A and a benzyl thioester at the C-terminus of the fragment B, or a combination of a thiophenyl ester at the C-terminus of the fragment A and a mercaptoethane sulfonyl ester at the C-terminus of the fragment B, etc. is preferable.

Moreover, the -SH groups in the respective N-terminal Cys residues of the fragments B to D may be protected with protective groups, if desired. For example, these -SH groups may be protected as thiazolidine. This protective group is deprotected at the desired point in time up to ligation reaction. For example, a protective group, such as a disulfide group, which is spontaneously deprotected under conditions where ligation occurs, may be used directly in ligation reaction shown below without being deprotected. The disulfide group is easily deprotected under reaction conditions of the subsequent ligation method.

(Step of linking by ligation method)

In the method for producing the oligosaccharide chain-added extracellular domain of AILIM according to the present invention, the solid-phase-synthesized fragments A and B are linked by the KCL method to prepare a fragment A + B. The fragments C and D are linked by the NCL method to prepare a fragment C + D. Finally, the fragment A + B and the fragment C + D are linked by the NCL method.

In the present specification, a fragment obtained by linking a fragment X and a fragment Y by a ligation method is referred to as a fragment X + Y.

Two peptide fragments are mixed in a solution such as a 100 mM phosphate buffer solution in the presence of a thiol catalyst such as 4-mercaptophenylacetic acid, benzylmercaptan, or thiophenol, as appropriate. Preferably, reaction is performed at a ratio of 0.5 to 2 equivalents of the second peptide fragment to 1 equivalent of the first peptide fragment using approximately 5 equivalents of the thiol catalyst. It is preferred that the reaction should be performed for approximately 1 to 30 hours under conditions involving a pH of approximately 6.5 to 7.5 and approximately 20 to 40°C. The progress of the reaction can be confirmed using approaches known in the art such as HPLC and MS in combination.

A reducing agent such as tris(2-carboxyethyl)phosphine hydrochloride (TCEP) is added thereto to inhibit side reaction. The resulting product can be purified, if desired, to thereby link the first peptide fragment and the second peptide fragment.

Moreover, the KCL method was reported by Kent and is a reaction-kinetically controlled NCL method (Kent et al., Angew. Chem. Int. Ed., 2006, 45, 3985-3988). In the NCL method, one of two fragments to be linked has a carboxyl group at the C-terminus while the C-terminus of the other fragment is thioesterified. In this method, the fragment is linked to the thioesterified C-terminus of the other fragment. By contrast, the KCL method can be used when the fragments to be linked are both thioesterified. Thus, a more highly eliminable thioester is subjected to linking reaction.

As described above, thiol groups generally have stronger reactivity in the following order: an arylthiol group, a benzyl-type thiol group, and an alkyl-type thiol group. Thus, a C-terminal thioester -C(=O)-SR wherein R is an aryl group is most highly eliminable, followed by that wherein R is a benzyl group and that wherein R is an alkyl group. Based on this fact, the thioester in each fragment can be determined to thereby link the fragments in the desired order. For example, one of the fragments may have a thiophenyl ester while the other fragment may have an ethyl thioester. In such a case, the thiophenyl ester is first subjected to linking reaction to obtain a peptide having the ethyl thioester at the C-terminus. Moreover, one of the fragments may have a 4-mercaptophenyl thioester (MPAA) while the other fragment may have a benzyl thioester. In such a case, MPAA is first reacted to obtain a peptide having the benzyl thioester at the C-terminus. Furthermore, one of the fragments may have a thiophenyl ester while the other fragment may have a mercaptoethane sulfonyl ester. In such a case, the thiophenyl ester is first reacted to obtain a peptide having the mercaptoethane sulfonyl ester at the C-terminus.
The peptide chain thus obtained by the KCL method has a thioester at the C-terminus. Therefore, this peptide chain can be used directly in ligation with another fragment.

(Folding step)

In the method for producing the oligosaccharide chain-added extracellular domain of AILIM of the present invention, a folding step may be performed after the linking of the fragments A to D to have the resulting polypeptide in an appropriate higher-order structure.
The folding step can be performed using various methods known in the art and can be performed, for example, by dialysis in a folding buffer. The folding buffer contains, for example, a compound having a guanidino group, such as guanidine, or a salt thereof, and may have a pH of 6.0 to 9.0. The dialysis may be performed several times. In this case, the composition or pH of the buffer may be the same or different among the dialysis treatments.
The folding of the polypeptide can be confirmed by an arbitrary method for analyzing the conformation of the polypeptide. Examples thereof include, but not limited to, a disulfide mapping method, evaluation on the avidity of a specific antibody to a conformational epitope, and X-ray analysis.

<Pharmaceutical composition>

A pharmaceutical composition of the present invention comprises the oligosaccharide chain-added extracellular domain of AILIM of the present invention as an active ingredient. The soluble oligosaccharide chain-added extracellular domain of AILIM of the present invention, when administered into an organism, competitively inhibits the interaction between AILIM expressed on T cell surface and its ligand, by virtue of high affinity for the ligand. Thus, the pharmaceutical composition of the present invention is effective for many diseases for which immunosuppression is therapeutically desirable. The pharmaceutical composition of the present invention is particularly effective for the treatment or prevention of an AILIM-related disease and, in particular, effective as immunosuppressive agents that regulates the AILIM pathway. Examples of main indications of the pharmaceutical composition of the present invention include autoimmune diseases such as idiopathic thrombocytopenic purpura (ITP), systemic lupus erythematosus (SLE), type I diabetes mellitus, multiple sclerosis, aplastic anemia, psoriasis, allergy, inflammatory biliary disease, and rheumatoid arthritis.
Moreover, examples of the indications of the pharmaceutical composition of the present invention also include transplantation of organs or tissues (e.g., kidney, heart, lung, bone marrow, skin, and cornea) and graft-versus-host disease (GvHD) in bone marrow transplantation. The GvHD is a generic term for conditions that occur by the attack on recipient organs as a result of activation of donor T cells by the recognition of alloantigens and signals from costimulatory receptors when donor-derived cells are transferred to the recipient. The pharmaceutical composition of the present invention is understood to block only signals from costimulatory molecules to induce immunological unresponsiveness (anergy) so that GvHD is inhibited.
The inhibition of GvHD can be analyzed experimentally, for example, by in *vitro* mixed lymphocyte reaction (MLR) through which T cells from a donor are cocultured with antigen-presenting cells derived from a donor differing in transplantation antigen therefrom. It has previously confirmed that soluble CTLA4 inhibits CD28-mediated signal transduction to thereby inhibit MLR and induce anergy (see Non Patent Literatures 3 and 4). In MLR using T cells activated by CD3 stimulation, soluble AILIM has been shown to have reaction inhibitory effect equivalent to that of soluble CTLA4 (Non Patent Literature 5). Thus, the pharmaceutical composition of the present invention is understood to exert further effect.
Further examples of the indications of the pharmaceutical composition of the present invention include asthma.
The pharmaceutical composition is formulated in a usual pharmaceutical composition form using diluents or excipients usually used, such as a filler, an extender, a binder, a wetting agent, a disintegrant, a surfactant, and a lubricant. Examples of such a pharmaceutical composition include tablets, pills, powders, solutions, suspension, emulsions, granules, capsules, suppositories, and injections.
The amount of the oligosaccharide chain-added extracellular domain of AILIM of the present invention contained

in the pharmaceutical composition is not particularly limited and can be selected appropriately from within a wide range. It is usually preferred that 1 to 70% by weight of the oligosaccharide chain-added extracellular domain of AILIM of the present invention should be contained in the pharmaceutical composition.

The pharmaceutical composition comprising the oligosaccharide chain-added extracellular domain of AILIM of the present invention as an active ingredient may further contain a different active ingredient or may be used in combination with a pharmaceutical composition containing the different active ingredient. Moreover, the pharmaceutical composition comprising the oligosaccharide chain-added extracellular domain of AILIM of the present invention as an active ingredient may further comprise one or more of different oligosaccharide chain-added extracellular domain(s) of AILIM of the present invention as an active ingredient or may be used in combination with a pharmaceutical composition comprising one or more of different oligosaccharide chain-added extracellular domain(s) of AILIM of the present invention as an active ingredient.

The administration method of the pharmaceutical composition according to the present invention is not particularly limited, and the pharmaceutical composition according to the present invention is administered by a method according to various dosage forms, the age and sex of a patient, the severity of disease, and the other conditions. Examples of the administration method for tablets, pills, solutions, suspensions, emulsions, granules, and capsules include oral administration. Moreover, injections can be administered intravenously, intramuscularly, intracutaneously, subcutaneously, or intraperitoneally either alone or as a mixture with a replacement fluid such as glucose or amino acids. Suppositories are intrarectally administered.

The dose of the pharmaceutical composition can be selected appropriately according to usage, the age and sex of a patient, the severity of disease, and the other conditions and is usually a dose that gives 0.1 to 900 nmol, preferably 1 to 90 nmol of the oligosaccharide chain-added extracellular domain of AILIM of the present invention per kg of body weight. The oligosaccharide chain-added extracellular domain of AILIM of the present invention has the much higher ability to bind to the ligand than that of the conventional soluble extracellular domain of AILIM (fusion protein of the extracellular domain of AILIM with IgG Fc) and is thus understood to achieve a smaller dose than that of the conventional soluble extracellular domain of AILIM.

The number of doses of the pharmaceutical composition can be selected appropriately according to usage, the age and sex of a patient, the severity of disease, and the other conditions and is, for example, 3 doses/day, 2 doses/day, 1 dose/day. Furthermore, the smaller number of doses (e.g., 1 dose/week or 1 dose/month) may be selected according to its stability in blood. Preferably, the number of doses of the pharmaceutical composition is 1 dose/day or a lower frequency. The oligosaccharide chain-added extracellular domain of AILIM of the present invention has the much higher ability to bind to the ligand than that of the conventional soluble extracellular domain of AILIM and is thus understood to achieve the smaller number of doses than that of the conventional soluble extracellular domain of AILIM.

The added oligosaccharide chain in the oligosaccharide chain-added extracellular domain of AILIM of the present invention is easily decomposed in the metabolic system in vivo. Moreover, in an aspect of the present invention, the oligosaccharide chain has a structure that is present in *vivo* as a glycopeptide (or glycoprotein) comprising the oligosaccharide chain bound to a peptide (or protein). Thus, the oligosaccharide chain-added extracellular domain of AILIM of the present invention and the pharmaceutical composition comprising the oligosaccharide chain-added extracellular domain of AILIM of the present invention as an active ingredient are unlikely to exhibit adverse reaction or antigenicity even when administered into an organism, and are advantageously in little danger of, for example, causing allergy reaction or losing drug efficacy due to antibody production.

Furthermore, the oligosaccharide chain-added extracellular domain of AILIM of the present invention can be supplied stably and conveniently in large amounts and is also very useful from the viewpoint of stably providing high-quality pharmaceuticals.

The present invention also provides a method for treating or preventing a disease related to the extracellular domain of AILIM, comprising administering an effective amount of the oligosaccharide chain-added extracellular domain of AILIM of the present invention.

The terms used in the present specification are used for illustrating particular embodiments and are not intended to limit the present invention.

Moreover, the term "comprising" used in the present specification means that described items (members, steps, factors, numbers, etc.) are present and the presence of the other items (members, steps, factors, numbers, etc.) is not excluded therefrom, unless the context evidently requires different interpretation.

All terms (including technical terms and scientific terms) used herein have the same meanings as those understood in a broad sense by those skilled in the art to which the present invention belongs, unless otherwise defined. The terms used herein should be interpreted as having meanings consistent with meanings in the present specification and related technical fields and should not be interpreted in an idealized or excessively formal sense, unless otherwise defined.

The embodiments of the present invention may be described with reference to a schematic diagram. However, such a schematic diagram may be exaggerated for the purpose of clear illustration.

Terms such as "first" or "second" are used for expressing various factors. However, these factors are understood to be not limited by these terms. These terms are used merely for differentiating one factor from the other factors. For example, the first factor may be described as the second factor, and vice versa, without departing from the scope of the present invention.

Hereinafter, the present invention will be described in more detail with reference to Examples. However, the present invention can be embodied in various aspects. Thus, the present invention is not intended to be limited to Examples described herein by any means.

Examples

According to the scheme shown in Figure 2, a 120-amino acid extracellular domain of AILIM containing an oligosaccharide chain added to a position corresponding to position 69 of SEQ ID NO: 7 (SEQ ID NO: 9) was divided into four fragments, which were each synthesized by an Fmoc solid-phase synthesis method and sequentially linked by an NCL method or a KCL method. These fragments are referred to as fragments A, B, C, and D in this order from the N-terminus. The fragment A corresponds to positions 1 to 42 of SEQ ID NO: 8; the fragment B corresponds to positions 43 to 62; the fragment C corresponds to positions 63 to 88; and the fragment D corresponds to positions 89 to 120.
As shown in the diagram, the fragments D and C were synthesized and linked by the NCL method after thioesterification of the C-terminal carboxyl group of the fragment C to obtain a fragment C + D. In this procedure, the N-terminal Cys of the fragment C was protected as thiazolidine, which was converted to Cys after the linking. Cys containing a protective group introduced in the thiol group was used in the solid-phase synthesis of the fragment D. Moreover, an oligosaccharide chain was added to position 7 of SEQ ID NO: 11 using asialo-nonasaccharide chain-added Asn in the solid-phase synthesis of the fragment C.
Moreover, the fragments A and B were also synthesized by the solid-phase synthesis method. The fragments A and B were linked to prepare a fragment A + B, which was then subjected to linking to the fragment C + D by the NCL method. Therefore, the respective C-terminal carboxyl groups of the fragments A and B were thioesterified so that the fragment A + B also had a thioester at the C-terminus. The fragment B had a propane thioester at C-terminus, and the fragment A had a more highly eliminable thiophenyl ester at the C-terminus. Cys containing a protective group introduced in the thiol group was used in the solid-phase synthesis of the fragment A.
Finally, the fragment A + B and the fragment C + D were linked by the NCL method, and the protective group in Cys was deprotected to obtain the oligosaccharide chain-added extracellular domain of AILIM of interest.
Hereinafter, each step will be described in detail.

<Synthesis of fragment D>

HMPA-PEGA resin (50 μmol) was used as a resin. Amino acid condensation was performed in the same way as in the conventional technique using an Fmoc method. Fmoc-Lys(Boc)-OH (117.1 mg, 0.25 mmol) was condensed with MSNT (74 mg, 0.25 mmol), N-methylimidazole (15 μl, 0.19 mmol), and DCM (1 ml). Subsequent amino acids were condensed with HOBt (33.8 mg, 0.25 mmol), DIPCI (38 μl, 0.25 mmol), and DMF (1 ml). The amino acids used were as follows:

> HMPA-PEGA resin (50 μmol) was used as a resin. Amino acid condensation was performed in the same way as in the conventional technique using an Fmoc method. Fmoc-Lys(Boc)-OH (117.1 mg, 0.25 mmol) was condensed with MSNT (74 mg, 0.25 mmol), N-methylimidazole (15 μl, 0.19 mmol), and DCM (1 ml). Subsequent amino acids were condensed with HOBt (33.8 mg, 0.25 mmol), DIPCI (38 μl, 0.25 mmol), and DMF (1 ml). The amino acids used were as follows: Leu (88.4 mg, 0.25 mmol), Gln (92.1 mg, 0.25 mmol), Cys (acm) (103.6 mg, 0.25 mmol), Ser (tBu) (95.9 mg, 0.25 mmol), Glu (OtBu) (106.4 mg, 0.25 mmol), Tyr (tBu) (115 mg, 0.25 mmol), Ile (88.4 mg, 0.25 mmol), His (trt) (154.9 mg, 0.25 mmol), Gly (74.3 mg, 0.25 mmol), Thr (tBu) (99.4 mg, 0.25 mmol), Val (84.9 mg, 0.25 mmol), Phe (96.8 mg, 0.25 mmol), Pro (84.4 mg, 0.25 mmol), Asp (OtBu) (102.9 mg, 0.25 mmol), Asn (trt) (149.2 mg, 0.25 mmol)

To the resin with 32 amino acid residues elongated, a 95% aqueous TFA solution was added, and the mixture was stirred at room temperature for 2 hours. The resin was filtered off, and the reaction solution was concentrated. The residue was purified by HPLC to obtain the peptide (SEQ ID NO: 10) of interest.
HPLC analysis: Cadenza CD-18 (3 mm, 4.6 x 75 mm)
Developing solvent A: 0.1% aqueous TFA solution, B: 0.1% TFA acetonitrile:water = 90:10, gradient A:B = 80:20 → 25:75, 15 min., flow rate: 1.0 ml/min.
HPLC purification: Vydac C-18 (5 mm, 10 x 250 mm), gradient A:B = 70:30 → 30:70, 25 min., flow rate: 4.0 ml/min.
ESI-MS: Calcd for $C_{172}H_{265}N_{41}O_{48}S_3$: $[M+2H]^{2+}$ 1886.7, found 1886.8

<Synthesis of fragment C>

Oligosaccharide chain-added Asn containing asialo-nonasaccharide chain shown below was used as an asialo oligosaccharide chain-added amino acid, and HMPB-PEGA resin (0.1 mmol) was used as a resin. Amino acid condensation was performed in the same way as in the conventional technique using an Fmoc method.

[Formula 10]

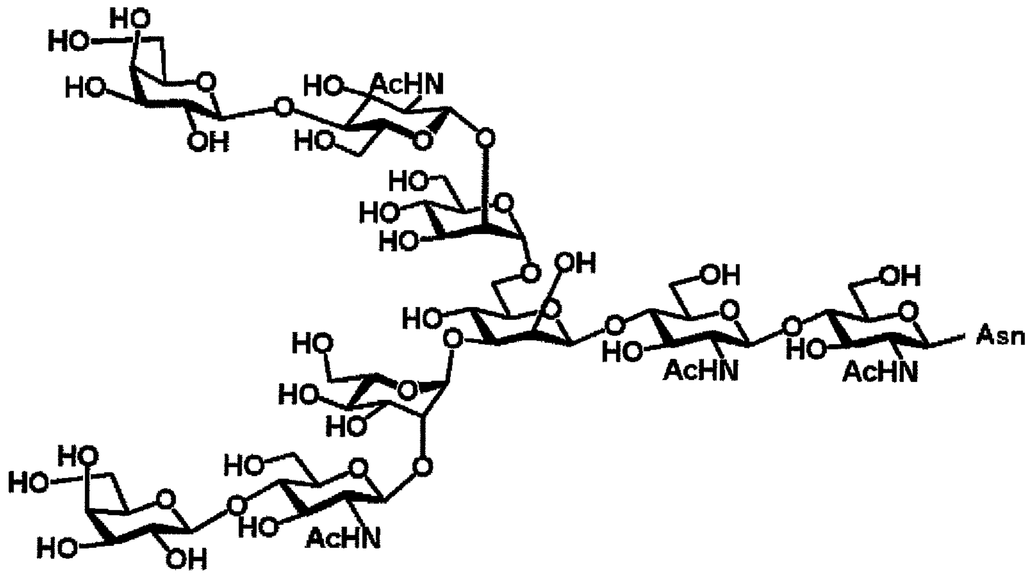

Fmoc-Phe-OH (193.7 mg, 0.5 mmol) was condensed with MSNT (148.2 mg, 0.5 mmol), N-methylimidazole (29.9 ml, 0.375 mmol), and DCM (2 ml). Subsequent amino acids were condensed with HOBt (67.6 mg, 0.5 mmol), DIPCI (77 ml, 0.5 mmol), and DMF (2 ml). The amino acids used were as follows:
Tyr (tBu) (229.8 mg, 0.5 mmol), Asn (177.2 mg, 0.5 mmol), Ala (155.7 mg, 0.5 mmol), His (trt) (309.9 mg, 0.5 mmol), Ser (tBu) (191.7 mg, 0.5 mmol), Asp (OtBu) (205.8 mg, 0.5 mmol), Leu (176.8 mg, 0.5 mmol), Phe (193.7 mg, 0.5 mmol), Val (169.7 mg, 0.5 mmol)

A 10 μmol aliquot of the resin with 19 amino acid residues elongated was transferred to an Eppendorf tube, and asialo oligosaccharide chain-added Asn (40 mg, 20 μmol) was condensed thereto. The same method as the conventional technique was used using DEPBT (8.98 mg, 30 μmol), DIPEA (3.4 μl, 40 μmol), and a DMF:DMSO (= 4:1) mixed solution (667 μl). Furthermore, amino acids were sequentially condensed to this asialo oligosaccharide chain-added peptide. Amino acids were condensed with HOBt (6.8 g, 50 μmol), DIPCI (7.7 μl, 50 μmol), and DMF (1.25 ml). The amino acids used were as follows:
Ser (tBu) (19.2 mg, 50 μmol), Leu (17.7 mg, 50 μmol), Gln (18.4 mg, 50 μmol), His (trt) (31 mg, 50 μmol), Boc-L-thiazolidine-4-carboxylicacid (11.7 mg, 50 μmol)

To this resin with 26 amino acid residues elongated, AcOH:TFE = 1:1 (3 ml) was added, and the mixture was reacted at room temperature for 18 hours. The resin was filtered off, and the reaction solution was concentrated. The excision procedure was repeated 3 times. This was purified by HPLC to obtain the asialo oligosaccharide chain-added peptide of interest containing protected side chains.
Subsequently, a thioester form of the fragment C having the asialo oligosaccharide chain was synthesized.
The protected fragment C having the asialo oligosaccharide chain (63 mg, 10 μmol), MS4A (10 mg), and benzylmercaptan (35.5 μl, 0.3 mmol) were stirred at -20°C for 1 hour in a DMF solvent (1.35 ml) under argon flow. Then, PyBOP (26 mg, 50 μmol) and DIPEA (8.5 μl, 50 μmol) were added thereto, and the mixture was stirred for 6 hours. Then, diethyl ether was added to the reaction solution to precipitate the compound. After filtration, the precipitate was collected using DMF,

and the filtrate was concentrated under reduced pressure. Then, a 95% aqueous TFA solution was added thereto, and the mixture was stirred at room temperature for 2 hours. The reaction solution was concentrated under reduced pressure, and this was purified by HPLC to obtain the thioester form of the fragment C of interest having the asialo oligosaccharide chain (SEQ ID NO: 11).
HPLC analysis: Cadenza CD-18 (3 mm, 4.6 x 75 mm)
Developing solvent A: 0.1% aqueous TFA solution, B: 0.1% TFA acetonitrile:water = 90:10, gradient A:B = 80:20 25:75, 15 min., flow rate: 1.0 ml/min.
HPLC purification: Vydac C-18 (5 mm, 10 x 250 mm), gradient A:B = 65:35 → 25:75, 20 min., flow rate: 3.0 ml/min.
ESI-MS: Calcd for $C_{211}H_{299}N_{41}O_{86}S_2$: $[M+H]^+$ 4848.0, found 4847.9 (deconvoluted)

<Linking of thioester form of fragment C to fragment D by NCL method>

The thioester form of the fragment C (1 mg, 0.21 μmol) and the fragment D (1.5 mg, 0.33 μmol) were transferred to an Eppendorf tube. 8 M guanidine hydrochloride/20 mM TCEP/50 mM MPAA/0.2 M phosphate buffer solution (165 ml) adjusted to pH 7.23 was added thereto, and the mixture was reacted at 37°C. The reaction was monitored by HPLC to confirm 4 hours later that the compound of interest (fragment C + D containing thiazolidine at the N-terminus; SEQ ID NO: 12) was formed and the thioester form of the fragment C as a starting material disappeared. Thus, a 0.2 M aqueous methoxyamine hydrochloride solution was added to the reaction solution until its pH became 4.0. The mixture was reacted at room temperature. 4 hours later, it was confirmed by HPLC and mass spectrometry that the N-terminal thiazolidine was converted to cysteine. Then, the compound was purified by HPLC to obtain the compound of interest (fragment C + D; SEQ ID NO: 13).
HPLC analysis: Vydac C-18 (5 mm, 4.6 x 75 mm), Developing solvent A: 0.1% aqueous TFA solution, B: 0.1% TFA acetonitrile:water = 90:10, gradient A:B = 70:30 → 25:75, 30 min., flow rate: 1.0 ml/min.
HPLC purification: Vydac C-18 (5 mm, 4.6 x 75 mm), gradient A:B = 65:35 → 20:80, 30 min., flow rate: 1.0 ml/min.
ESI-MS: Calcd for $C_{375}H_{556}N_{82}O_{134}S_4$: $[M+5H]^{5+}$ 1698.0, found 1698.2

<Synthesis of fragment B>

HMPB-PEGA resin (0.1 mmol) was used as a resin. Amino acid condensation was performed in the same way as in the conventional technique using an Fmoc method. Fmoc-Phe-OH (193.7 mg, 0.5 mmol) was condensed with MSNT (148.2 mg, 0.5 mmol), N-methylimidazole (29.9 ml, 0.375 mmol), and DCM (2 ml). Subsequent amino acids were condensed with HOBt (67.6 mg, 0.5 mmol), DIPCI (77 ml, 0.5 mmol), and DMF (2 ml). The amino acids used were as follows:
Lys (Boc) (234.3 mg, 0.5 mmol), Leu (176.7 mg, 0.5 mmol), Ser (tBu) (191.7 mg, 0.5 mmol), Ile (176.7 mg, 0.5 mmol), Val (169.7 mg, 0.5 mmol), Thr (tBu) (198.8 mg, 0.5 mmol), Asn (177.2 mg, 0.5 mmol), Gly (148.7 mg, 0.5 mmol), Asp (OtBu) (205.8 mg, 0.5 mmol), Boc-L-thiazolidine-4-carboxylic acid (116.6 mg, 0.5 mmol)
A 15 μmol aliquot of the resin with 20 amino acid residues elongated was collected. 1% TFA (in DCM) (3 ml) was added thereto, and the mixture was stirred at room temperature for 2 minutes. The resin was filtered off, and the filtrate was added dropwise into 10% pyridine (5 ml). This procedure was repeated 5 times. The reaction solution was concentrated, and water was then added to the concentrate to precipitate the compound of interest. After filtration, the precipitate was collected using DMF, and the filtrate was concentrated under reduced pressure. A 20-residue peptide containing protected side chains was obtained.
This protected peptide (54 mg, 20 μmol), MS4A, and 1-propanethiol (54.4 μl, 0.6 mmol) were stirred at -20°C for 1 hour in a DMF solvent (2.7 ml) under argon flow. Then, PyBOP (52.3 mg, 0.1 mmol) and DIPEA (17.0 μl, 0.1 mmol) were added thereto, and the mixture was stirred for 3 hours. Then, diethyl ether was added to the reaction solution to precipitate the compound. After filtration, the precipitate was collected using DMF, and the filtrate was concentrated under reduced pressure. Then, a 95% aqueous TFA solution was added thereto, and the mixture was stirred at room temperature for 2 hours. The reaction solution was concentrated under reduced pressure. Then, the mixture was dissolved in an 8 M guanidine buffer (200 mM sodium phosphate, pH = 6.5). Subsequently, a 0.2 M aqueous methoxyamine solution was added thereto to adjust the pH of the reaction solution to around 4.0. After stirring for 6 hours, the compound was purified by HPLC to obtain the peptide of interest containing a propane thioester (SEQ ID NO: 14).
HPLC analysis: Cadenza CD-18 (3 mm, 4.6 x 75 mm) Developing solvent A: 0.1% aqueous TFA solution, B: 0.1% TFA acetonitrile:water = 90:10, gradient A:B = 80:20 25:75, 15 min., flow rate: 1.0 ml/min.
HPLC purification: Vydac C-18 (5 mm, 10 x 250 mm), gradient A:B = 65:35 → 25:75, 20 min., flow rate: 3.0 ml/min.
ESI-MS: Calcd for $C_{95}H_{165}N_{25}O_{29}S_2$: $[M+2H]^{2+}$ 1093.8, found 1093.7

<Synthesis of fragment A>

A microwave method was used in the solid-phase synthesis of the fragment A. HMPB-PEGA resin (0.1 mmol) was used as a resin. Amino acid condensation was performed in the same way as in the conventional technique using an Fmoc method. Fmoc-Leu-OH (176.7 mg, 0.5 mmol) was condensed with MSNT (148.2 mg, 0.5 mmol), N-methylimidazole (29.9 ml, 0.375 mmol), and DCM (2 ml). Subsequent amino acids were condensed with HOBt (67.6 mg, 0.5 mmol), DIPCI (77 ml, 0.5 mmol), and DMF (2 ml). The amino acids used were as follows:
Ile (176.7 mg, 0.5 mmol), Gln (184.2 mg, 0.5 mmol), Gly (148.7 mg, 0.5 mmol), Lys (Boc) (234.3 mg, 0.5 mmol), Leu (176.7 mg, 0.5 mmol), Met (185.7 mg, 0.5 mmol), Phe (193.7 mg, 0.5 mmol), Val (169.7 mg, 0.5 mmol), Asp (OtBu) (205.7 mg, 0.5 mmol), Pro (168.7 mg, 0.5 mmol),
Tyr (tBu) (229.8 mg, 0.5 mmol), Cys (acm) (206.6 mg, 0.5 mmol), Asn (177.2 mg, 0.5 mmol), His (Trt) (309.9 mg, 0.5 mmol), Ala (155.7 mg, 0.5 mmol), Ser (tBu) (191.7 mg, 0.5 mmo1)

A 20 μmol aliquot of the resin with 42 amino acid residues elongated was collected, and 1% TFA (in DCM:TFE = 1:1) was added thereto. To remove the Fmoc group, the mixture was irradiated with microwaves (Discover 1 (manufactured by CEM Corp.)) at 20 W at 37°C for 3 minutes. The resin was filtered off, and the filtrate was added dropwise into hexane. The procedures from the addition of 1% TFA to the dropwise addition into hexane were repeated 3 times. The reaction solution was concentrated to obtain a 42-residue peptide containing protected side chains.
This protected peptide (153.2 mg, 20 μmol), MS4A, and thiophenol (61.4 μl, 0.6 mmol) were stirred at -20°C for 1 hour in a DMF solvent (2.7 ml) under argon flow. Then, PyBOP (52.0 mg, 0.1 mmol) and DIPEA (17.0 μl, 0.1 mmol) were added thereto, and the mixture was stirred for 3 hours. Then, diethyl ether was added to the reaction solution to precipitate the compound. After filtration, the precipitate was collected using DMF, and the filtrate was concentrated under reduced pressure. Then, a 95% aqueous TFA solution was added thereto, and the mixture was stirred at room temperature for 2 hours. The reaction solution was concentrated under reduced pressure, and this was purified by HPLC to obtain the peptide of interest containing a thiophenyl ester (SEQ ID NO: 15). HPLC analysis: Cadenza CD-18 (3 mm, 4.6 x 75 mm) Developing solvent A: 0.1% aqueous TFA solution, B: 0.1% TFA acetonitrile:water = 90:10, gradient A:B = 80:20 25:75, 15 min., flow rate: 1.0 ml/min.
HPLC purification: Vydac C-18 (5 mm, 10 x 250 mm), gradient A:B = 70:30 → 30:70, 20 min., flow rate: 3.0 ml/min.
ESI-MS: Calcd for $C_{112}H_{161}N_{27}O_{31}S_{21}$ : M+4H] $^{4+}$1238.4, found 1238.3

<Linking of fragment A and fragment B by KCL>

The phenyl thioester form of the fragment A (1 mg, 0.20 μmol) and the ethyl thioester form of the fragment B (2 mg, 0.91 μmol) were transferred to an Eppendorf tube. 8 M guanidine hydrochloride/40 mM TCEP/0.2 M phosphate buffer solution (500 μl) adjusted to pH 6.85 was added thereto, and the mixture was reacted at room temperature. The reaction was monitored by HPLC to confirm 2 hours later that the compound of interest was formed. Then, the compound was purified by HPLC to obtain the compound of interest (fragment A + B; SEQ ID NO: 16).
HPLC analysis: Vydac C-18 (5 mm, 4.6 x 75 mm), Developing solvent A: 0.1% aqueous TFA solution, B: 0.1% TFA acetonitrile:water = 90:10, gradient A:B = 70:30 → 25:75, 30 min., flow rate: 1.0 ml/min.
HPLC purification: Vydac C-18 (5 mm, 4.6 x 75 mm), gradient A:B = 65:35 → 20:80, 30 min., flow rate: 1.0 ml/min.
ESI-MS: Calcd for $C_{369}H_{546}N_{80}O_{132}S_4$: [M+3H] $^{3+}$2353.8, found 2353.8

<Linking of fragment A + B and fragment C + D by NCL method>

The thioester form of the fragment A + B (SEQ ID NO: 17; 1 mg, 0.21 μmol) and the fragment C + D (SEQ ID NO: 13; 1 mg, 0.12 μmol) were transferred to an Eppendorf tube. 8 M guanidine hydrochloride/40 mM TCEP/50 mM MPAA/0.2 M phosphate buffer solution (200 μl) adjusted to pH 6.85 was added thereto, and the mixture was reacted at 37°C. The reaction was monitored by HPLC to confirm 12 hours later that the compound of interest (fragment A + B + C + D; SEQ ID NO: 17) was formed. Then, the compound was purified by gel-filtration chromatography to obtain the compound of interest.
HPLC purification: gel filtration on Superdex 75 (TM) 10/300 mm GL. The column (10 x 300 mm) was equilibrated as follows: 10% (v/v) formic acid, flow rate: 0.4 mL/min. ESI-MS: Calcd for $C_{688}H_{1055}N_{163}O_{223}S_8$: [M+7H] $^{7+}$2205.9, found 2206.3, [M+8H] $^{8+}$1930.3, found 1930.6, [M+9H] $^{9+}$1715.9, found 1716.3, [M+10H] $^{10+}$1544.4, found 1544.7, [M+11H] $^{11+}$1404.1, found 1404.6, [M+12H] $^{12+}$1287.2, found 1287.4, [M+13H] $^{13+}$1188.2, found 1188.6, [M+14H] $^{14+}$1103.4, found 1103.8, [M+15H] $^{15+}$1030.0, found 1030.2, [M+16H] $^{16+}$965.6, found 966.0

<Deprotection of Acm group in Cys of fragment A + B + C + D>

90% acetic acid (140 μl) and AgOAc (1.4 μmol, 0.3 mg) were added to the fragment A + B + C + D (1 mg, 0.07

μmol), and the mixture was reacted at room temperature. 8 hours later, DTT (2 mg) was added thereto, and the mixture was reacted for 1 hour. Then, it was confirmed by gel-filtration chromatography that the compound of interest (oligosaccharide chain-added extracellular domain of AILIM (hereinafter, referred to as "synthetic AILIM"); SEQ ID NO: 9) was formed. Then, the compound was purified on Superdex to obtain the compound of interest.
HPLC purification: gel filtration on Superdex 75 (TM) 10/300 mm GL. The column (10 x 300 mm) was equilibrated as follows: 10% (v/v) formic acid, flow rate: 0.4 mL/min. ESI-MS: Calcd for $C_{679}H_{1040}N_{160}O_{220}S_8$: $[M+7H]^{7+}$2175.4, found 2175.4, $[M+8H]^{8+}$1903.6, found 1903.4, $[M+9H]^{9+}$1692.2, found 1692.0, $[M+10H]^{10+}$1523.1, found 1523.1, $[M+11H]^{11+}$1384.7, found 1384.7, $[M+12H]^{12+}$1269.4, found 1269.2, $[M+13H]^{13+}$1171.8, found 1171.7, $[M+14H]^{14+}$1088.2, found 1088.0, $[M+15H]^{15+}$1015.7, found 1015.6, $[M+16H]^{16+}$952.3, found 952.3

<Folding step>

After freeze drying, the fragment A + B + C + D was dissolved in 2 ml of a buffer (6 M guanidine hydrochloride, 0.1 M TRIZMA base) adjusted to pH 8.5. A 1 ml aliquot of the solution was added to each of dialysis tubes a; Spectra/Por 7 (Funakoshi Co., Ltd., MWCO: 8000, 7.5 mm x 12 mm x 2 cm) and b; Spectra/Por (Funakoshi Co., Ltd., MWCO: 8000, 32 mm x 50 mm x 6 cm), and the dialysis tubes a and b were tightly closed. The contents in each of the dialysis tubes a and b were dialyzed overnight in 500 ml of a buffer (3 M guanidine hydrochloride, 0.1 M TRIZMA base, 20 μM cysteine, 2.5 μm cystine) adjusted to pH 8.5.
After the overnight dialysis, the contents in each of the dialysis tubes a and b were transferred to 500 ml of a buffer (1 M guanidine hydrochloride, 0.1 M TRIZMA base) adjusted to pH 8.0, and dialyzed again. 8 hours later, the contents in each of the dialysis tubes a and b were transferred to 500 ml of a buffer (10 mM PBS) adjusted to pH 7.0, and dialyzed overnight. After the overnight dialysis, the solution in each dialysis tube was collected and concentrated using Amicon (Millipore Corp., MWCO: 5000). A portion of each sample concentrated using Amicon (Millipore Corp., MWCO: 5000) was substituted by a 1% aqueous formic acid solution using Amicon. The resulting solutions were used as evaluation samples.

<Evaluation of synthetic AILIM>

(1) Analysis of binding reactivity between AILIM and ICOS ligand (B7h)

The AILIM molecule whose expression is induced on activated T cells transmits costimulatory signals into the cell through its binding to the AILIM ligand molecule B7h/B7H2/B7RP-1. The binding reactivity between synthetic AILIM and the B7h molecule was analyzed for the purpose of evaluating the biological functions of synthetic AILIM.
Dilution series of each evaluation sample solution of synthetic AILIM were added to an ELISA plate, and each well was coated therewith by reaction at 37°C for 2 hours. After the reaction, the sample solution was discarded, D-PBS (-) containing 0.4% bovine serum albumin and 0.05% Tween 20 (blocking solution) was added at a concentration of 150 μL/well, and each well was blocked by reaction at 37°C for 1 hour. The plate thus blocked was washed 3 times with D-PBS (-) containing 0.1% bovine serum albumin and 0.05% Tween 20 (washing buffer). An AILIM ligand B7-H2/Fc Chimera (R&D Systems, Inc.) biotinylated using EZ-Link NHS-PEO Solid Phase Biotinylation Kit was added at a concentration of 200 ng/50 μL/well and reacted at 37°C for 1.5 hours. Unreacted ligand molecules were removed by washing 3 times with the washing buffer.
Subsequently, streptavidin-labeled horseradish peroxidase (BD-Pharmingen) diluted 1000-fold with D-PBS (-) containing 0.1% bovine serum albumin was added at a concentration of 100 μL/well and reacted at 37°C for 1 hour. After the reaction, the plate was washed with 3 times with the washing buffer to remove unreacted streptavidin-labeled horseradish peroxidase. A mixed solution containing the same amounts of ABTS Peroxidase Substrate (KPL) and Peroxidase Solution B ($H_2O_2$) (KPL) was added at a concentration of 100 μL/well and reacted at room temperature for 30 minutes in the dark. The reaction was terminated by the addition of 1% SDS at a concentration of 100 μL/well, and the OD value at 405 nm was measured using a plate reader (VersaMax, Molecular Devices, LLC).
An ELISA plate was coated with Recombinant human ICOS/Fc Chimera (R&D Systems, Inc.; hereinafter, referred to as "ICOS/Fc") as a positive control substance or CTLA-4/Fc Chimera (R&D Systems, Inc.) as a negative control substance, and the same procedures as above were performed. This ICOS/Fc is an expressed fusion protein of the extracellular domain of human AILIM with human IgGl via a polypeptide linker and has a structure shown below. The human AILIM moiety contained oligosaccharide chains added to the same 3 positions as in the wild type.

[Formula 11]

| Human **AILIM (Glu 21 - Phe 141)** | **IEGRMD** | Human **$IgG_1$ (Pro 100 - Lys 330)** |
|---|---|---|

The results are shown in Figure 3A. ICOS/Fc as a positive control exhibited binding reactivity with the ligand in a manner dependent on the amount of its coating. Synthetic AILIM exhibited ligand binding activity even in the lower amount of its coating than that of the positive control in both the lot. a and the lot. b.

To compare the activities of the positive control and synthetic AILIM, a concentration dependence curve was prepared (Figure 3B) from the results about ICOS/Fc to obtain the following approximate expression:

$$y = 0.0123e^{12.842x}$$

From this expression, an ICOS/Fc concentration necessary for obtaining the same absorbance as that of synthetic AILIM (lot. a) was calculated. The results are shown in Table 5.

[Table 5]

| A: Synthetic AILIM concentration (nmol/ml) | O.D.450 | B: Value on ICOS/Fc calibration curve (nmol/ml) | B/A |
|---|---|---|---|
| 0.06684 | 0.321267 | 0.761512 | 11.39306 |
| 0.01671 | 0.163967 | 0.101014 | 6.045123 |
| 0.004178 | 0.086 | 0.037115 | 8.884412 |

As is evident from the values of B/A, an ICOS/Fc concentration approximately 10 times that of synthetic AILIM was required for obtaining ligand binding activity equivalent to that of synthetic AILIM.

Unexpectedly, synthetic AILIM exhibited such a high ability to bind to its ligand, compared to the wild type. This is probably because synthetic AILIM has a simple structure, compared with ICOS/Fc, and can thus bind to the ligand without undergoing steric hindrance in the binding region.

(2) Analysis of reactivity with anti-ICOS monoclonal antibody

Next, the presence or absence of anti-ICOS antibody epitope formation in the synthetic AILIM molecule was studied by antibody reactivity analysis for the purpose of analyzing the higher-order structure of synthetic AILIM.

Dilution series of each synthetic AILIM sample solution were added to an ELISA plate, and each well was coated therewith by reaction at 37°C for 2 hours. After the reaction, the sample solution was discarded, D-PBS (-) containing 0.4% bovine serum albumin and 0.05% Tween 20 (blocking solution) was added at a concentration of 150 μL/well, and each well was blocked by reaction at 37°C for 1 hour. The plate thus blocked was washed 3 times with D-PBS (-) containing 0.1% bovine serum albumin and 0.05% Tween 20 (washing buffer). An anti-AILIM antibody Mouse Anti-ICOS (BD-Pharmingen) adjusted to 10 μg/mL with D-PBS (-) containing 0.1% bovine serum albumin was added at a concentration of 50 μL/well and reacted at 37°C for 1.5 hours. Unreacted antibodies were removed by washing 3 times with the washing buffer. Subsequently, horseradish peroxidase-labeled anti-mouse Ig (H + L) antibody (Vector Laboratories, Inc.) diluted 1000-fold with D-PBS (-) containing 0.1% bovine serum albumin was added as an antibody for detection at a concentration of 100 μL/well and reacted at 37°C for 1 hour. After the reaction, the plate was washed with 3 times with the washing buffer to remove unreacted horseradish peroxidase-labeled anti-mouse Ig (H + L) antibody. A mixed solution containing the same amounts of ABTS Peroxidase Substrate (KPL) and Peroxidase Solution B ($H_2O_2$) (KPL) was added at a concentration of 100 μL/well and reacted at room temperature for 30 minutes in the dark. The reaction was terminated by the addition of 1% SDS at a concentration of 100 μL/well, and the OD value at 405 nm was measured using a plate reader (VersaMax, Molecular Devices, LLC). An ELISA plate was coated with Recombinant human ICOS/Fc Chimera (R&D Systems, Inc.) as a positive control substance or CTLA-4/Fc Chimera (R&D Systems, Inc.) as a negative control substance, and the same procedures as above were performed.

The results are shown in Figure 4. Recombinant human ICOS/Fc Chimera as a positive control exhibited antibody reactivity in a manner dependent on the amount of its coating and contained an antibody epitope formed in the molecule. By contrast, synthetic AILIM seemed to neither exhibit antibody reactivity nor form an antibody epitope.

Sequence Listing Free Text

SEQ ID NO: 1 represents human AILIM protein.
SEQ ID NO: 2 represents the amino acid sequence of rat AILIM1 protein.
SEQ ID NO: 3 represents the amino acid sequence of rat AILIM2 protein.
SEQ ID NO: 4 represents the amino acid sequence of mouse AILIM protein.
SEQ ID NO: 5 represents the amino acid sequence of human CD28 protein.
SEQ ID NO: 6 represents the amino acid sequence of human CTLA4 protein.
SEQ ID NO: 7 represents the amino acid sequence of the extracellular domain of human AILIM.
SEQ ID NO: 8 represents the amino acid sequence of a 120-amino acid extracellular domain of human AILIM.
SEQ ID NO: 9 represents the amino acid sequence of a 120-amino acid extracellular domain of human AILIM containing an oligosaccharide chain added to a position corresponding to position 69 of SEQ ID NO: 7.
SEQ ID NO: 10 represents the amino acid sequence of a fragment D containing Cys 28 and Cys 29 with protected side chains.
SEQ ID NO: 11 represents the amino acid sequence of an oligosaccharide chain-added fragment C containing a thiazolidine-protected N-terminus and a thioesterified C-terminus.
SEQ ID NO: 12 represents the amino acid sequence of an oligosaccharide chain-added fragment C + D containing a thiazolidine-protected N-terminus and Cys 54 and Cys 55 with protected side chains.
SEQ ID NO: 13 represents the amino acid sequence of an oligosaccharide chain-added fragment C + D containing Cys converted at the N-terminus and Cys 54 and Cys 55 with protected side chains.
SEQ ID NO: 14 represents the amino acid sequence of a fragment B containing a thioesterified C-terminus.
SEQ ID NO: 15 represents the amino acid sequence of a fragment A containing Cys 22 with a protected side chain and a thioesterified C-terminus.
SEQ ID NO: 16 represents the amino acid sequence of a fragment A + B containing Cys 22 with a protected side chain and a thioesterified C-terminus.
SEQ ID NO: 17 represents the amino acid sequence of an oligosaccharide chain-added fragment A + B + C + D containing Cys 22, Cys 116, and Cys 117 with protected side chains.

## Claims

1. An oligosaccharide chain-added extracellular domain of AILIM, comprising an extracellular domain of AILIM which comprises an oligosaccharide chain bound to a position corresponding to position 69 in the amino acid sequence of the extracellular domain of human AILIM represented by SEQ ID NO: 7, and no oligosaccharide chain added to a position corresponding to position 3 or 90 therein.

2. An oligosaccharide chain-added extracellular domain of AILIM, comprising an extracellular domain of AILIM which comprises an oligosaccharide chain bound only to a position corresponding to position 69 in the amino acid sequence of the extracellular domain of human AILIM represented by SEQ ID NO: 7.

3. The oligosaccharide chain-added extracellular domain of AILIM according to claim 1 or 2, wherein the oligosaccharide chain is an oligosaccharide chain represented by

[Formula 1]

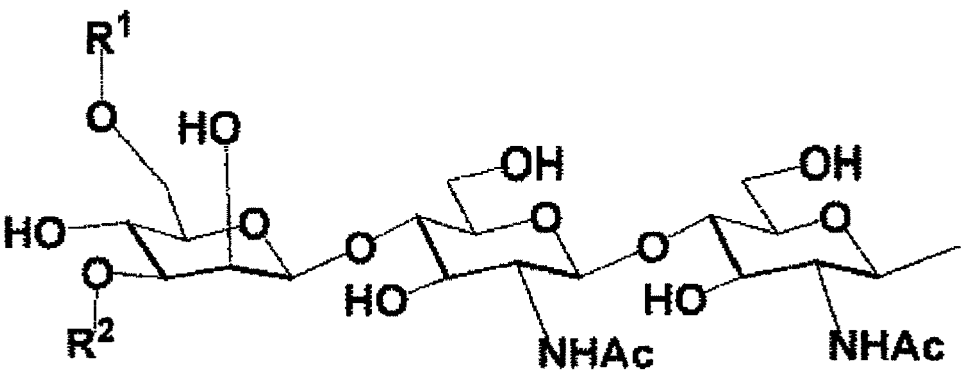

wherein $R^1$ and $R^2$, which are the same or different, each represent

[Formula 2]

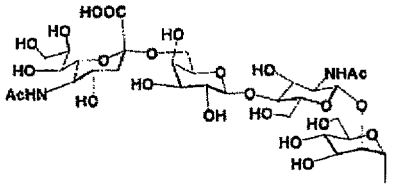
,
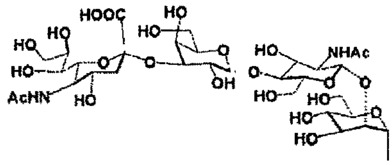
,
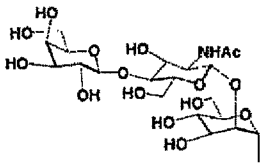
,
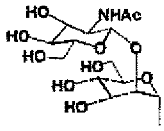
,
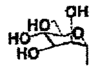
or
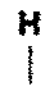

and Ac represents an acetyl group.

**4.** An oligosaccharide chain-added extracellular domain of AILIM, comprising an extracellular domain of AILIM which consists of the amino acid sequence represented by SEQ ID NO: 7, the extracellular domain comprising the following asialo-nonasaccharide chain bound only to Asn 69 of SEQ ID NO: 7:

[Formula 3]

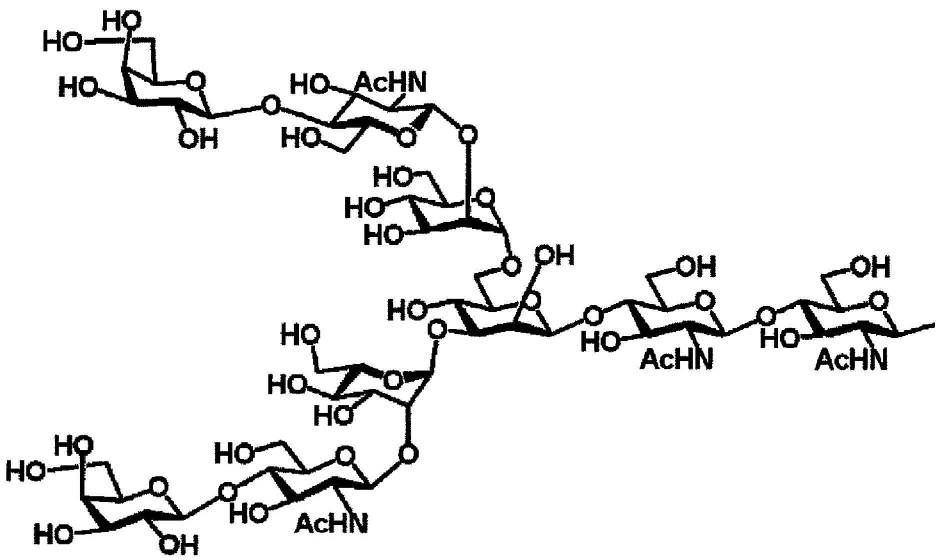

**5.** The oligosaccharide chain-added extracellular domain according to any one of claims 1 to 4, wherein the oligosaccharide chain is substantially homogeneous.

**6.** A method for producing an oligosaccharide chain-added extracellular domain of AILIM, comprising the steps of:

dividing the oligosaccharide chain-added extracellular domain of AILIM into the fragment A corresponding to a region of positions 1 to 42 in the amino acid sequence of the extracellular domain of human AILIM represented by SEQ ID NO: 7, the fragment B corresponding to a region of positions 43 to 62, the fragment C corresponding to a region of positions 63 to 88 and comprising an oligosaccharide chain-added position corresponding to position 69, and the fragment D corresponding to a region of position 89 to the C-terminus; and
preparing the fragments A to D, comprising:

introducing a protective group to the thiol group of Cys corresponding to position 22 in the fragment A; adding an oligosaccharide chain to Asn corresponding to position 69 in the fragment C; and introducing a protective group to the respective thiol groups of Cys 116 and Cys 117 in the fragment D;

thioesterifying the respective C-termini of the fragments A to C;
linking the fragments A and B by a KCL method;
linking the fragments C and D by an NCL method; and
linking the fragments A and B thus linked and the fragments C and D thus linked by the NCL method.

**7.** The method according to claim 6, wherein the preparation of the fragment A is performed by a solid-phase synthesis method, wherein in the solid-phase synthesis method, Cys having a protective group introduced to the thiol group is used as the Cys corresponding to position 22 in the amino acid sequence of the extracellular domain of human AILIM represented by SEQ ID NO: 7.

**8.** The method according to claim 6, wherein the preparation of the fragment C is performed by a solid-phase synthesis method, wherein in the solid-phase synthesis method, oligosaccharide chain-added asparagine is used as the Asn corresponding to position 69 in the amino acid sequence of the extracellular domain of human AILIM represented by SEQ ID NO: 7.

**9.** The method according to claim 6, wherein the preparation of the fragment D is performed by a solid-phase synthesis method, wherein in the solid-phase synthesis method, Cys having a protective group introduced to the thiol group is used as each of the Cys 116 and the Cys 117 in the amino acid sequence of the extracellular domain of human AILIM represented by SEQ ID NO: 7.

**10.** The method according to any one of claims 7 to 9, wherein the solid-phase synthesis method is an Fmoc solid-phase synthesis method.

**11.** The method according to claim 7, wherein the Fmoc solid-phase synthesis method comprises microwave irradiation in amino acid condensation reaction and/or Fmoc group elimination reaction.

**12.** The method according to any one of claims 6 to 11, wherein the oligosaccharide chain is an oligosaccharide chain represented by

[Formula 4]

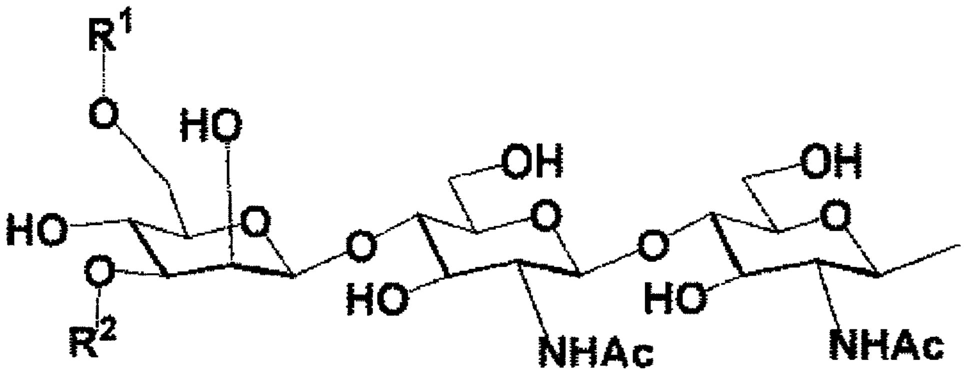

wherein $R^1$ and $R^2$, which are the same or different, each represent

[Formula 5]

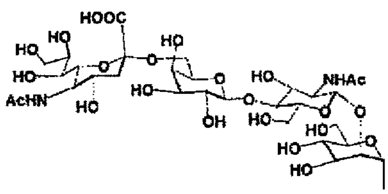,
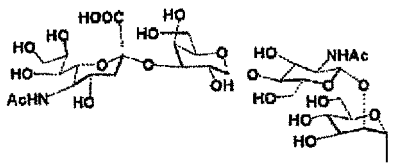,
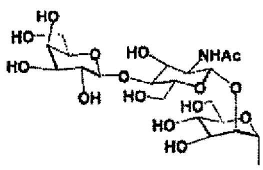,
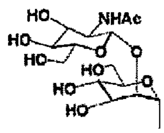,
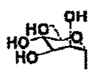 or 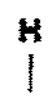

and Ac represents an acetyl group.

**13.** The method according to claim 12, wherein the oligosaccharide chain is the following asialo-nonasaccharide chain:

[Formula 6]

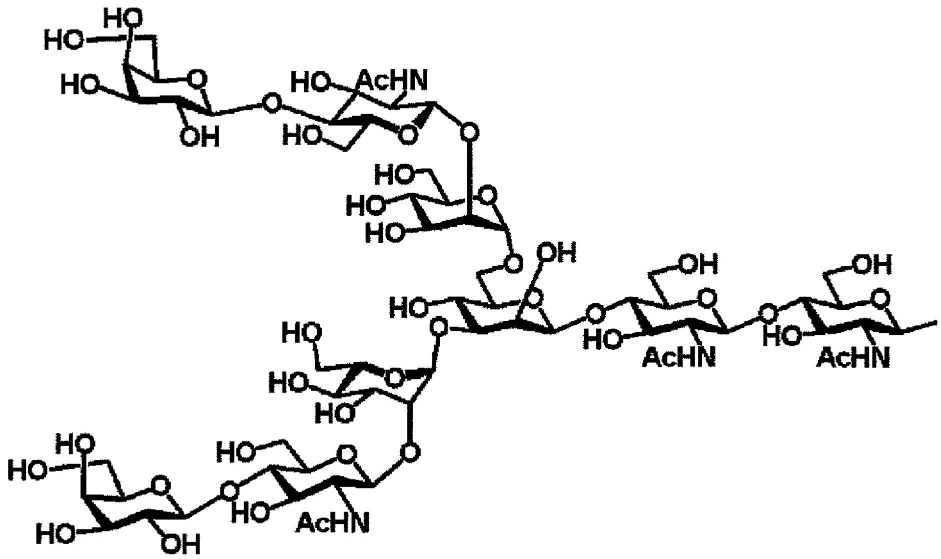

**14.** An oligosaccharide chain-added extracellular domain of AILIM produced by a method according to any one of claims 6 to 13, wherein the oligosaccharide chain is substantially homogeneous.

**15.** A pharmaceutical composition comprising an oligosaccharide chain-added extracellular domain of AILIM according to any one of claims 1 to 5 and 14 as an active ingredient.

**16.** The pharmaceutical composition according to claim 15, wherein the pharmaceutical composition is intended for the treatment or prevention of an AILIM-related disease.

**17.** The pharmaceutical composition according to claim 15, wherein the pharmaceutical composition is an immunosuppressive agent.

**18.** An immunosuppression method comprising administering an oligosaccharide chain-added extracellular domain of AILIM according to any one of claims 1 to 5 or a pharmaceutical composition according to any one of claims 15 to 17.

[Figure 1]

```
rat    AILIM-1   M--------K  PYFS------  -CVFVFCFL-  IKLLTGELND  LANHRMFSFH   34
rat    AILIM-2   M--------K  PYFS------  -CVFVFCFL-  IKLLTGELND  LANHRMFSFH   34
mouse  AILIM     M--------K  PYFC------  -HVFVFCFL-  IRLLTGEING  SADHRMFSFH   34
human  AILIM     M--------K  SGLW------  -YFFLFCLR-  IKVLTGEING  SANYEMFIFH   34
human  CD28      M--------L  RLLL------  -ALNLFP-S-  IQVTGNKILV  KQSPMLVAYD   33
human  CTLA4     MACLGFQRHK  AQLNLAARTW  PCTLLFFLLF  IPVFCKAMHV  AQPAVVLASS   50
Consensus        M--------K  ....------  -..F.FC.L-  I..LTGE.N.  .A...MF.FH   50

rat    AILIM-1   DG--GVQISC  NYPETVQQLK  MQLFK--DRE  V-LCDLTKTK  GSGNTVSIKN   79
rat    AILIM-2   DG--GVQISC  NYPETVQQLK  MQLFK--DRE  V-LCDLTKTK  GSGNTVSIKN   79
mouse  AILIM     NG--GVQISC  KYPETVQQLK  MRLFR--ERE  V-LCELTKTK  GSGNAVSIKN   79
human  AILIM     NG--GVQILC  KYPDIVQQFK  MQLLK--GGQ  I-LCDLTKTK  GSGNTVSIKS   79
human  CD28      NAV-NLSCKY  SYNLFSREFR  ASLHKGLDSA  VEVCVVYGNY  SQQLQVYSKT   82
human  CTLA4     RGIASFVCEY  ASPGKATEVR  VTVLRQADSQ  V-TEVCAATY  MTGNELTFLD   99
Consensus        .G--GVQI.C  .YP..VQQ.K  M.L.K--D..  V-LC.LTKTK  GSGN.VSIK.  100

rat    AILIM-1   PMSCPYQLSN  NSVSFFLDNA  DSSQGSYFLC  SLSIFDPPPF  QEKNLSGGYL  129
rat    AILIM-2   PMSCPYQLSN  NSVSFFLDNA  DSSQGSYFLC  SLSIFDPPPF  QEKNLSGGYL  129
mouse  AILIM     PMLCLYHLSN  NSVSFFLNNP  DSSQGSYYFC  SLSIFDPPPF  QERNLSGGYL  129
human  AILIM     LKFCHSQLSN  NSVSFFLYNL  DHSHANYYFC  NLSIFDPPPF  K-VTLTGGYL  128
human  CD28      GFNCDGKLGN  ESVTFYLQNL  YVNQTDIYFC  KIEVMYPPPY  LDNEKSNGTI  132
human  CTLA4     DSICTGTSSG  NQVNLTIQGL  RAMDTGLYIC  KVELMYPPPY  Y-LGIGNGTQ  148
Consensus        ...C...LSN  NSVSFFL.N.  D.SQ..YY.C  .LSIFDPPPF  ....LSGGYL  150

rat    AILIM-1   L-IYESQLCC  QLKLWLPVGC  --AAFVAALL  FGC-IFIVWF  AKKKYRSSVH  175
rat    AILIM-2   L-IYESQLCC  QLKLWLPVGC  --AAFVAALL  FGC-IFIVWF  AKKKYRSSVH  175
mouse  AILIM     H-IYESQLCC  QLKLWLPVGC  --AAFVVVLL  FGC-ILIIWF  SKKKYGSSVH  175
human  AILIM     H-IYESQLCC  QLKFWLPIGC  --AAFVVVCI  LGC-ILICWL  TKKKYSSSVH  174
human  CD28      IHVKGKHLCP  SPLFPGPSKP  FWVLVVVGGV  LACYSLLVTV  AFIIFWVRSK  182
human  CTLA4     IYVIDPEPCP  DSDFLLWIL-  --AAVSSGLF  FYSFLLTAVS  LSKMLKKRSP  195
Consensus        .-IYESQLCC  QLK.WLP.GC  --AAFV..L.  FGC-ILI.W.  .KKKY.SSVH  200

rat    AILIM-1   DP---NSEYM  FMAAVNTNKK  SRLAGTAPLR  ALGRGEHSSC  QDRN        216
rat    AILIM-2   DP---NSEYM  FMAAVNTNKK  SRLAGMTS--  ----------  ----        200
mouse  AILIM     DP---NSEYM  FMAAVNTNKK  SRLAGVTS--  ----------  ----        200
human  AILIM     DP---NGEYM  FMRAVNTAKK  SRLTDVTL--  ----------  ----        199
human  CD28      RSRLLHSDYM  NMTPRRPGPT  RKHYQPYAPP  ------RDFA  AYRS        220
human  CTLA4     LT---TGVYV  KMPPTEPECE  KQFQPYFIP-  ----------  --IN        223
Consensus        DP---NSEYM  FM.AVNT.KK  SRL......-  ----------  --..        244
```

[Figure 2]

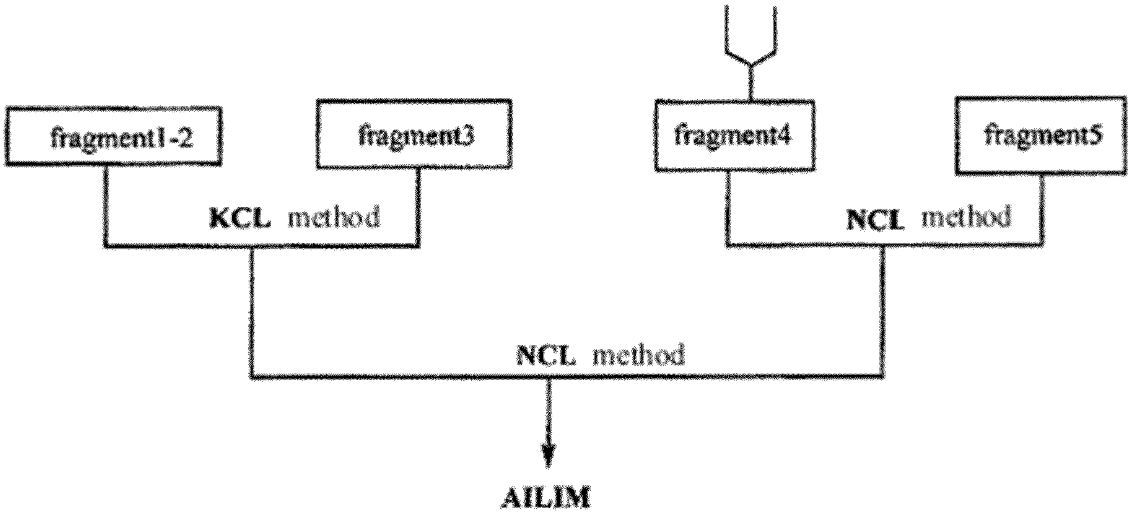


[Figure 3A]

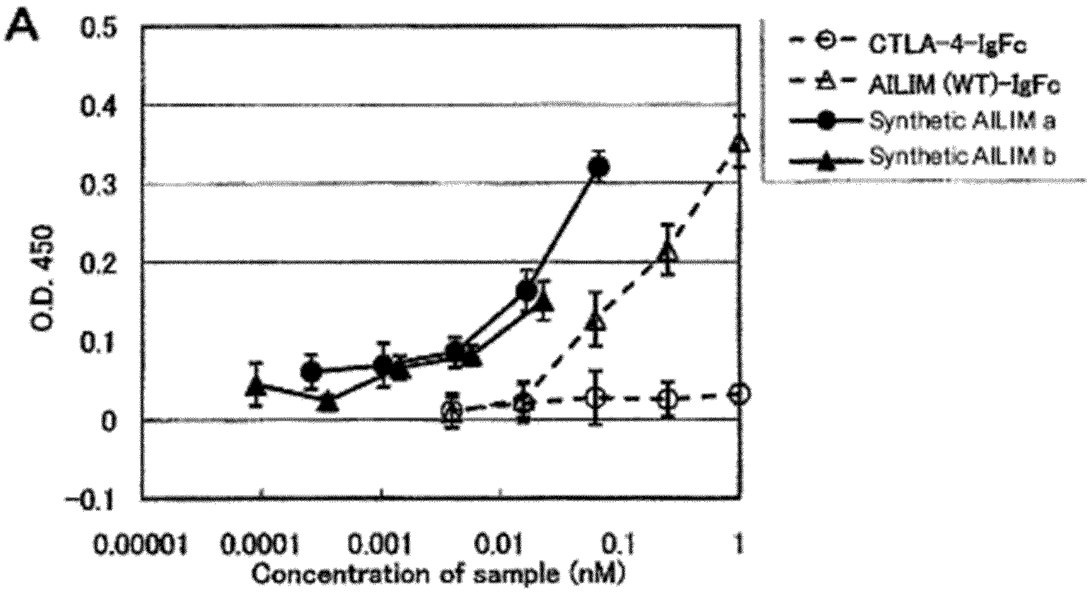

[Figure 3B]

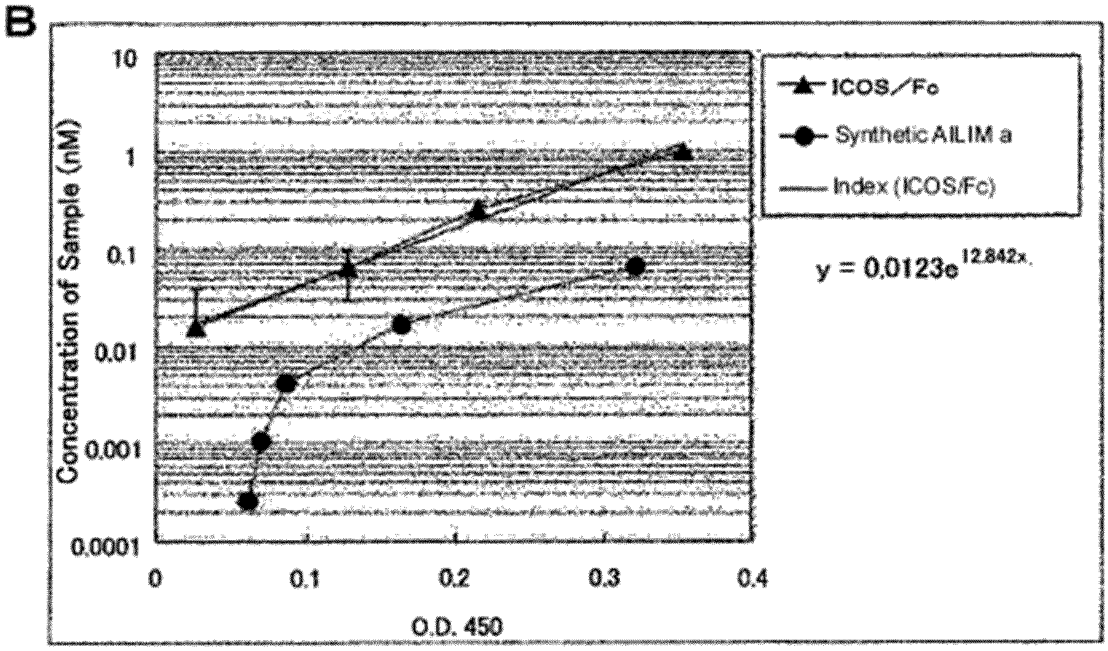


[Figure 4]

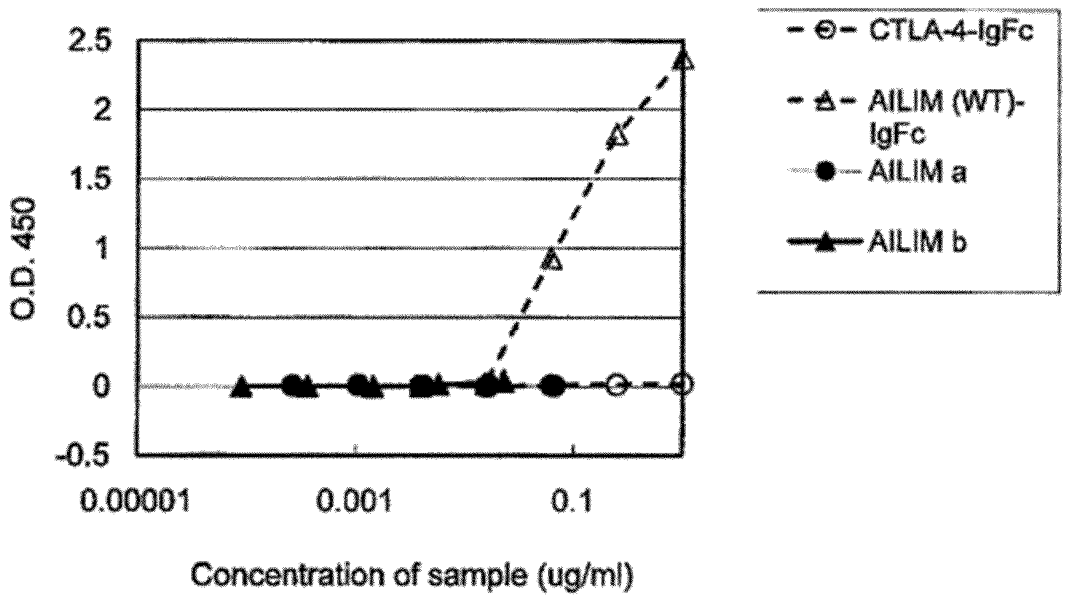

[Figure 5]

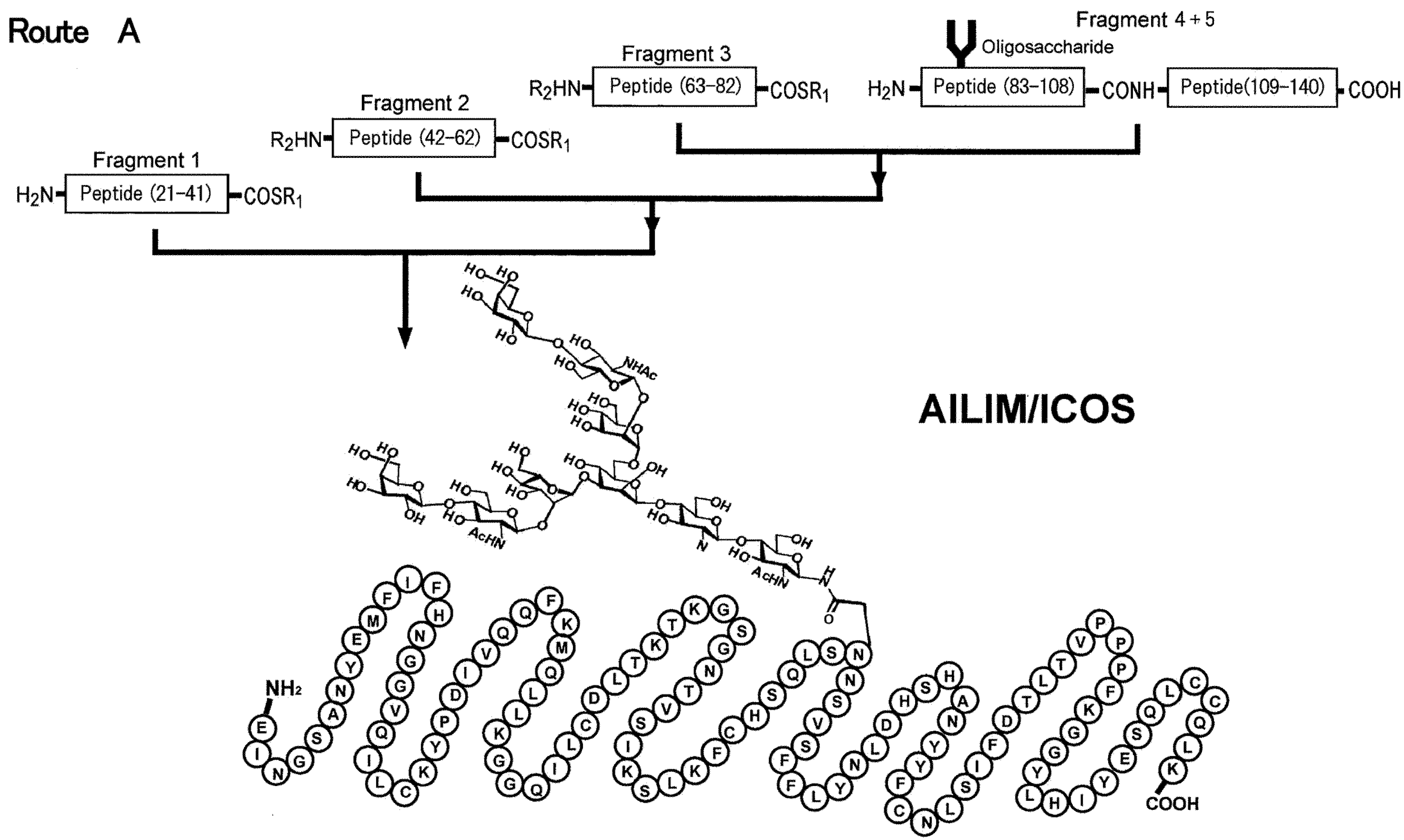
Route A
Fragment 1
Peptide (21-41)
Fragment 2
Peptide (42-62)
Fragment 3
Peptide (63-82)
Fragment 4 + 5
Oligosaccharide
Peptide (83-108)
Peptide(109-140)
AILIM/ICOS

[Figure 6]

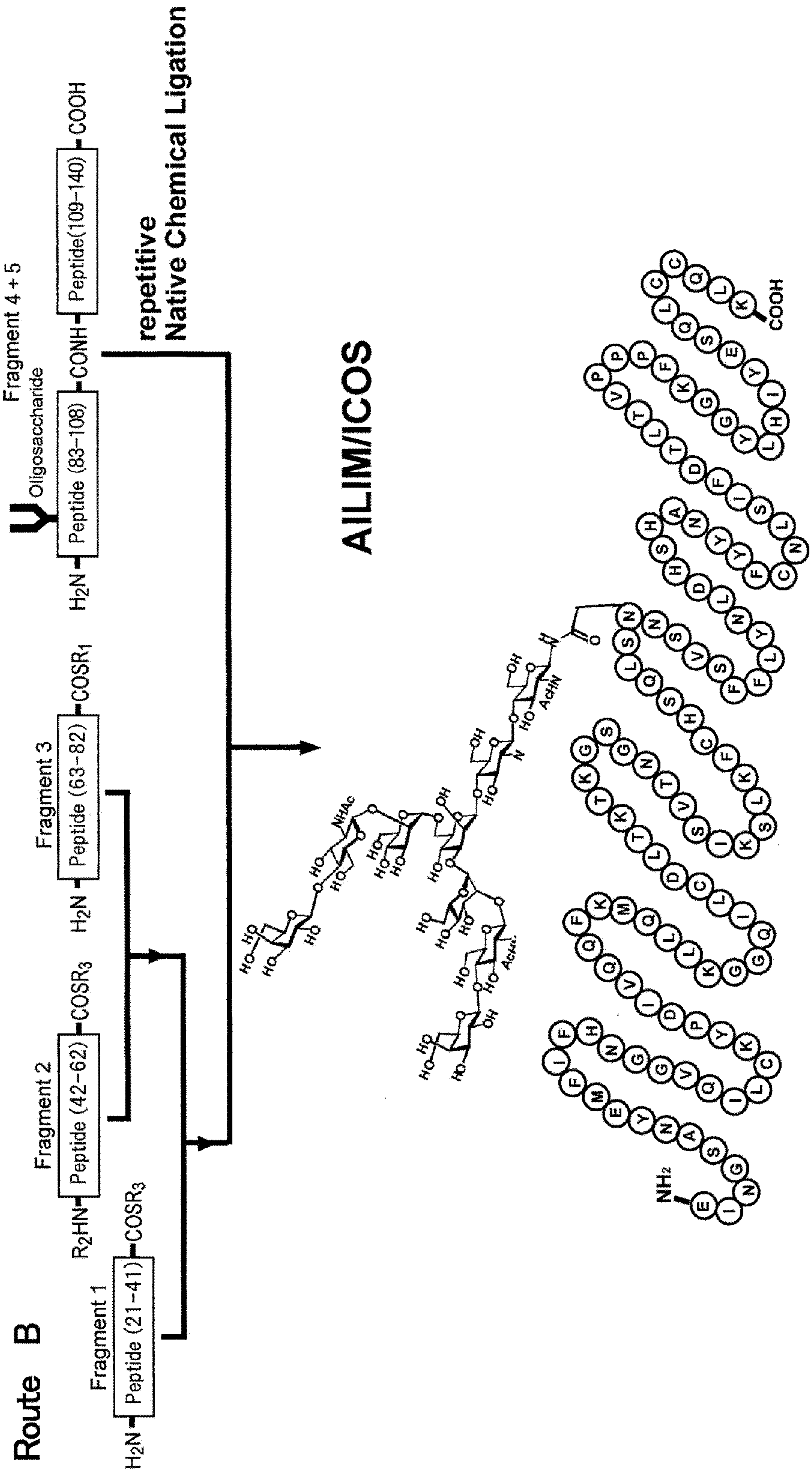
Route B
Fragment 1
$H_2N$-Peptide (21-41)-$COSR_3$
Fragment 2
$R_2HN$-Peptide (42-62)-$COSR_3$
Fragment 3
$H_2N$-Peptide (63-82)-$COSR_1$
Fragment 4 + 5
Oligosaccharide
$H_2N$-Peptide (83-108)-CONH-Peptide(109-140)-COOH
repetitive
Native Chemical Ligation
AILIM/ICOS

[Figure 7]

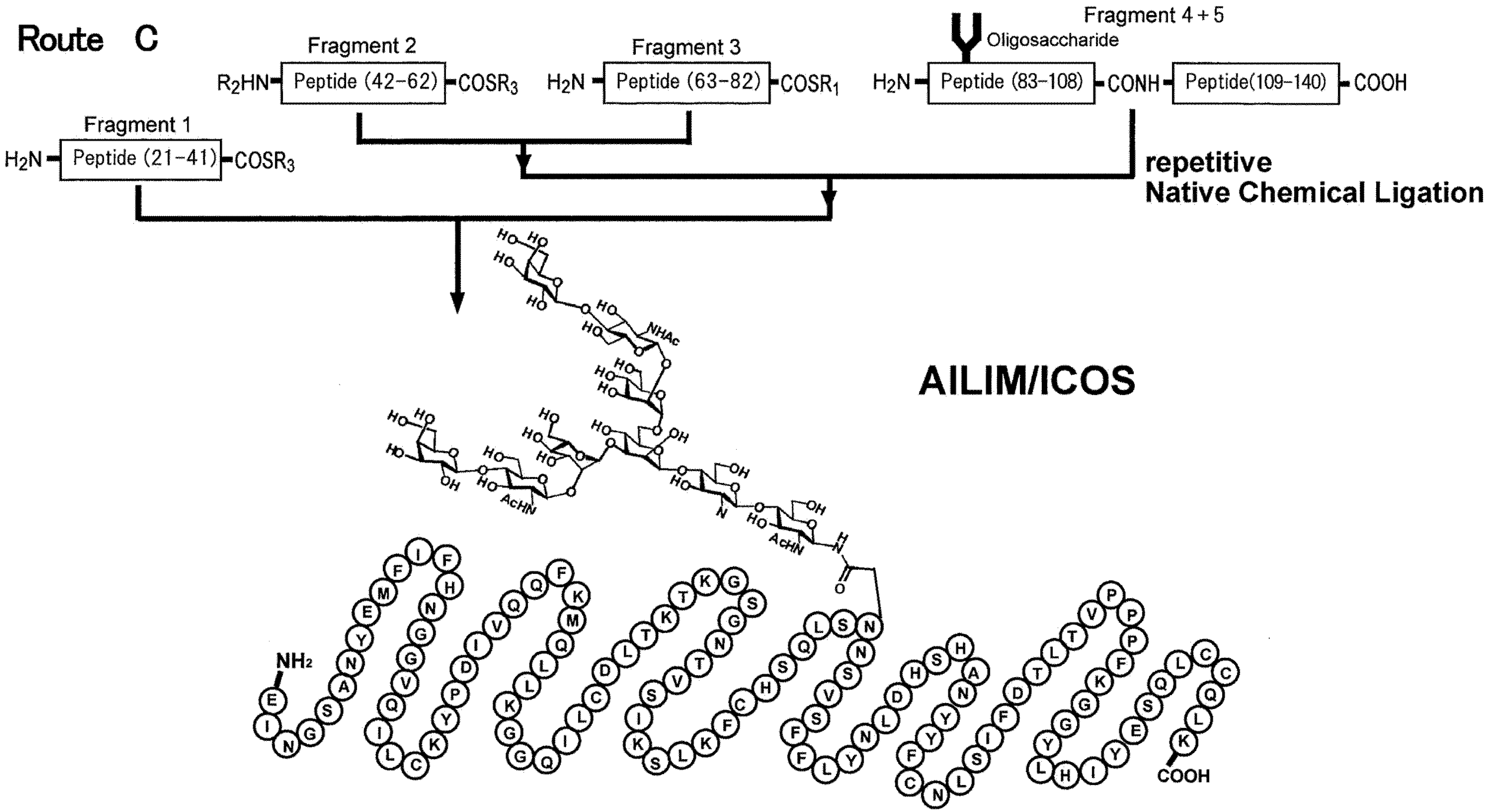
Route C
Fragment 1
H2N - Peptide (21-41) - COSR3
Fragment 2
R2HN - Peptide (42-62) - COSR3
Fragment 3
H2N - Peptide (63-82) - COSR1
Fragment 4 + 5
Oligosaccharide
H2N - Peptide (83-108) - CONH - Peptide(109-140) - COOH
repetitive
Native Chemical Ligation
AILIM/ICOS

[Figure 8]

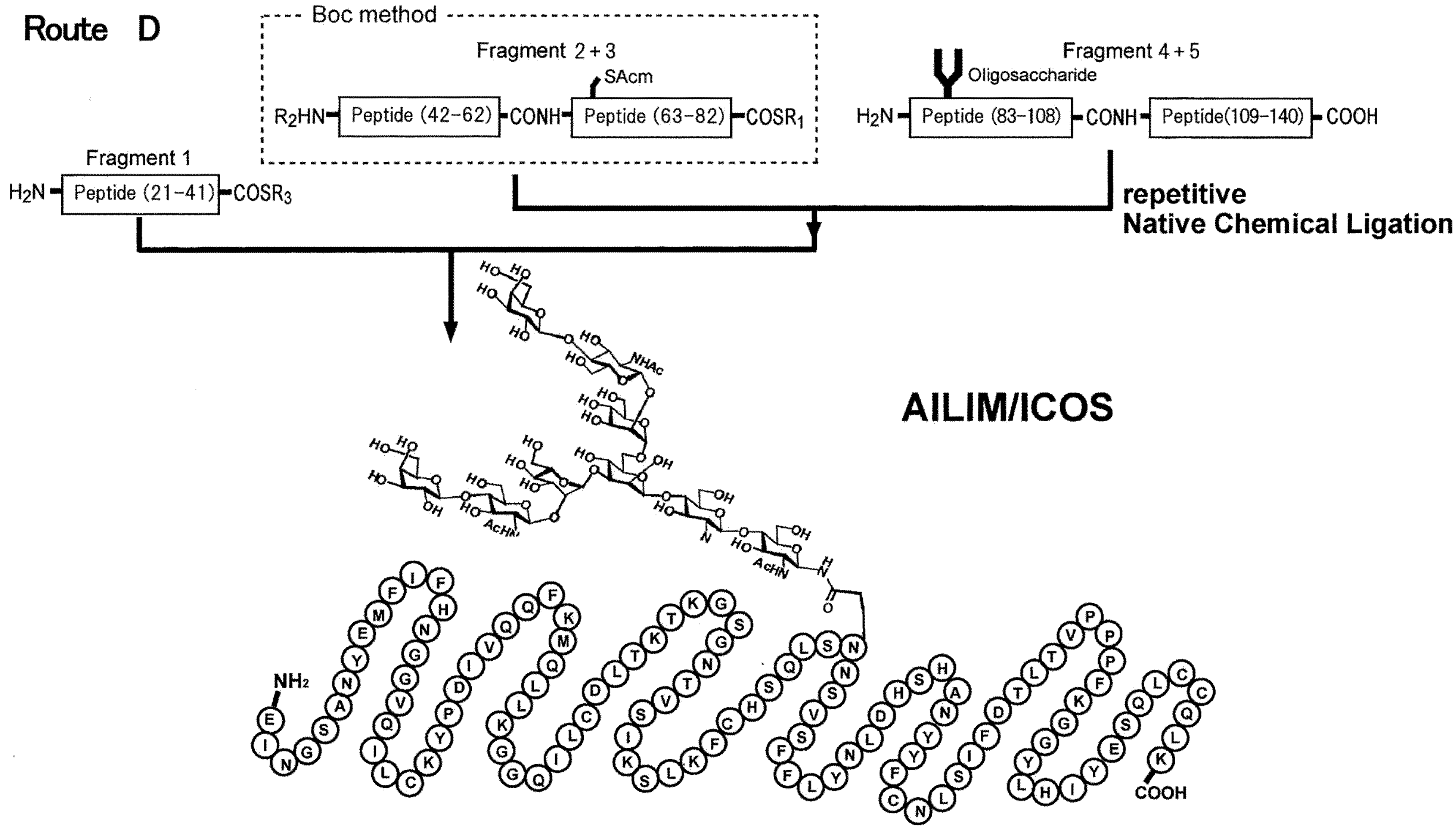
Route D
Boc method
Fragment 2 + 3
Fragment 1
Fragment 4 + 5
Oligosaccharide
Peptide (21-41)
Peptide (42-62)
Peptide (63-82)
Peptide (83-108)
Peptide(109-140)
repetitive
Native Chemical Ligation
AILIM/ICOS

**INTERNATIONAL SEARCH REPORT**

International application No.
PCT/JP2010/061749

A. CLASSIFICATION OF SUBJECT MATTER
*C07K14/705*(2006.01)i, *A61K38/00*(2006.01)i, *A61P37/06*(2006.01)i, *A61P43/00*(2006.01)i

According to International Patent Classification (IPC) or to both national classification and IPC

B. FIELDS SEARCHED

Minimum documentation searched (classification system followed by classification symbols)
C07K14/705, A61K38/00, A61P37/06, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/BIOSIS/MEDLINE/WPIDS(STN), JSTPlus/JMEDPlus/JST7580(JDreamII), PubMed

C. DOCUMENTS CONSIDERED TO BE RELEVANT

| Category* | Citation of document, with indication, where appropriate, of the relevant passages | Relevant to claim No. |
|---|---|---|
| X/Y | Akiko YOSHIHARA, Yasuhiro KAJIHARA, "Kyoshigeki To Tanpakushitsu no Gosei Kenkyu", CSJ: The Chemical Society of Japan Koen Yokoshu, 2007.03, vol.87th, no.2, page 1231, entire text | 1-5,14/6-13, 15-17 |
| Y | YU, V. et al., Convergent Chemical Synthesis and Crystal Structure of a 203 Amino Acid "Covalent Dimer" HIV-1 Protease Enzyme Molecule., Angew. Chem. Int. Ed., 2007, Vol.46, P.1667-1670, entire text, particularly, Scheme 1 | 6-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 August, 2010 (02.08.10) | 10 August, 2010 (10.08.10) |
| Name and mailing address of the ISA/ Japanese Patent Office | Authorized officer |
| Facsimile No. | Telephone No. |

Form PCT/ISA/210 (second sheet) (July 2009)

INTERNATIONAL SEARCH REPORT

International application No.
PCT/JP2010/061749

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-277293 A (Japan Tobacco Inc.), 02 October 2003 (02.10.2003), example 2 & US 2004/0253229 A1 & EP 1374901 A1 & WO 2002/070010 A1 & DE 60217839 A1 & CN 1518458 A | 15-17 |
| A | MURASE, T. et al., Efficient and systematic synthesis of a small glycoconjugate library having human complex type oligosaccharides., Carbohydr. Res., 2009.02, Vol.344, P.762-770, entire text | 1-17 |
| A | Naoki KAMEI et al., "T-saibo Kyoshigeki Bunshi AILIM/ICOS ni Okeru N-ketsugogata Tosa no Kino to Kozo no Kaiseki", Journal of Japanese Biochemical Society, 2007, 2P-0022, entire text | 1-5,14 |
| A | BANG, D. et al., Kinetically Controlled Ligation for the Convergent Chemical Synthesis of Proteins, Angew. Chem. Int. Ed., 2006, Vol.45, P.3985-3988, entire text | 6-13 |

Form PCT/ISA/210 (continuation of second sheet) (July 2009)

**INTERNATIONAL SEARCH REPORT**

International application No.
PCT/JP2010/061749

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 18
because they relate to subject matter not required to be searched by this Authority, namely:
The invention set forth in claim 18 involve methods for treatment of the human body by prevention or therapy and thus relates to a subject matter on which the International Searching Authority is not required to carry out a search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9838216 A
- WO 9634878 A
- JP 2003277293 A
- WO 03008431 A
- US 2004181054 A1
- WO 2004058984 A
- US 2006228784 A
- WO 2004058824 A
- US 2006009421 A1
- WO 2004070046 A
- US 2006205039 A1
- WO 2007011055 A
- WO 2004005330 A
- US 2005222382 A1
- US 2006228784 A1
- US 6184344 B


### Non-patent literature cited in the description


- **Hutloff A et al.** *Nature,* 1999, vol. 397, 263-266
- **Yoshinaga SK et al.** *Nature,* 1999, vol. 402, 827-832
- **Swallow MM et al.** *Immunity,* 1999, vol. 11, 423-432
- **Ling V et al.** *J Immunol,* 2000, vol. 164, 1653-1657
- **Sakamoto S et al.** *HYBRIDOMA AND HYBRIDOMICS,* 2001, vol. 20, 293-303
- **Tan P et al.** *J Exp Med,* 1993, vol. 177, 165-173
- **Gribben JG et al.** *Blood,* 1996, vol. 87, 4887-4893
- **Aicher A et al.** *J Immunol,* 2000, vol. 164, 4689-4696
- **Bang D et al.** *Angew. Chem. Int.,* 2006, vol. 45, 3985-3988
- *FEBS LETTERS,* February 1975, vol. 50 (3
- **Bacsa B. et al.** *J. Org. Chem.,* 2008, vol. 73, 7532-7542
- *J. Am. Chem. Soc.,* 1999, vol. 121, 11369-11374
- *Angew. Chem.,* 2005, vol. 44, 1650-1654
- **Muralidharan V et al.** *Nature Methods,* 2006, vol. 3 (6), 429-438
- **Kent et al.** *Angew. Chem.,* 2006, vol. 45, 3985-3988